# EUROPÄISCHE PATENTSCHRIFT

(11) **EP 1 651 645 B1**
(45) Veröffentlichungstag und Bekanntmachung des Hinweises auf die Patenterteilung: **21.02.2007**
(21) Anmeldenummer: 04741132.7
(22) Anmeldetag: 19.07.2004
(51) Int. Cl.: C07D 453/02

(54) **N-BIARYLAMIDE**
N-BIARYLAMIDES
N-BIARYLAMIDES

(30) Priorität: 30.07.2003 DE 10334724
(43) Veröffentlichungstag der Anmeldung: 03.05.2006
(73) Patentinhaber: Bayer HealthCare AG, 51368 Leverkusen (DE)
(72) Erfinder: FLESSNER, Timo, 42113 Wuppertal (DE); BÖSS, Frank-Gerhard, Berkshire SL5 7DF (GB); HAFNER, Frank-Thorsten, 42115 Wuppertal (DE); LUITHLE, Joachim, 42489 Wülfrath (DE); METHFESSEL, Christoph, 42327 Wuppertal (DE); TELAN, Leila, 42115 Wuppertal (DE)
(86) Internationale Anmeldenummer: PCT/EP2004/008037
(87) Internationale Veröffentlichungsnummer: WO 2005/012299

(56) Entgegenhaltungen:
- WO-A-03/078431
- DE-A- 10 162 442
- US-A- 5 998 429

## Beschreibung

Die Erfindung betrifft N-Biarylamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi und Changeux, *Neuropharmacol.* **1995,** *34,* 563-582). Ein funktioneller nAChR besteht aus fünf Untereinheiten, die unterschiedlich (bestimmte Kombinationen von α1-9 und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee und Role, *Annu. Rev. Physiol.,* **1995,** 57, 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte wie z.B. die Stimulierung von nAChR in der Muskulatur vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani and Levin, *Biol. Psychiatry* **2001,** *49,* 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., *J. Neurosci.* **1993,** *13,* 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide und Leslie, *Mol. Neurobiol.* **1999,** *20,* 1-16).

Bestimmte Chinuclidincarbonsäureanilide sind als Antiarrhythmika und Lokalanästhetika beschrieben (vgl. beispielsweise FR 1.566.045, GB 1 578 421 und Oppenheimer et al., *Life Sci.* **1991**, *48*, 977-985).

Die WO 01/60821 offenbart Biarylcarbonsäureamide mit Affinität zum α7-nAChR zur Behandlung von Lern- und Wahrnehmungsstörungen.

Die WO 03/043991, WO 03/055878 und WO 04/013136 offenbaren Chinuclidinamin-Derivate und die WO 03/051874, WO 03/078431 und DE 10162442.5 offenbaren Chinuclidinsäure-Derivate, die sich als α7-nAChR Agonisten zur Behandlung von Lern- und Wahrnehmungsstörungen eignen.

Die vorliegende Erfindung betrifft Verbindungen der Formel 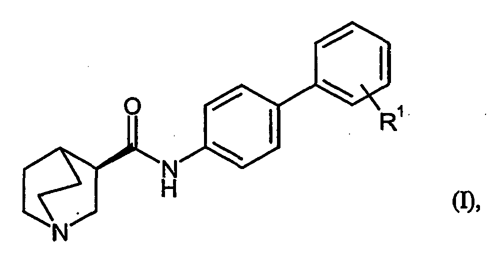 in welcher
- R¹: eine Gruppe der Formel -NR²-CO-NR³R⁴, -NR²-CO-CO-OR⁵, -NH-SO₂-R⁶, -SO₂NHR⁷ oder -NH-CO-R⁸, wobei
R² Wasserstoff oder C₁-C₆-Alkyl,
R³ und R⁴ unabhängig voneinander Wasserstoff, C₁-C₆-Alkyl, C₃-C₈-Cycloalkyl oder Phenyl,
welches gegebenenfalls mit bis zu 3 Resten unabhängig voneinander ausgewählt aus der Gruppe Halogen, Cyano, C₁-C₆-Alkyl, C₁-C₆-Alkoxy, Trifluormethyl und Trifluormethoxy substituiert ist, oder
R³ und R⁴ zusammen mit dem Stickstoffatom, an dem sie gebunden sind,
ein 5- bis 6-gliedriges Heterocyclyl bilden,
R⁵ Wasserstoff, C₁-C₆-Alkyl, C₃-C₈-Cycloalkyl oder Aryl, wobei C₁-C₆-Alkyl gegebenenfalls mit Aryl substituiert ist,
R⁶ C₁-C₆-Alkyl, C₃-C₈-Cycloalkyl, 5- bis 6-gliedriges Heterocyclyl, Aryl oder 5- bis 6-gliedriges Heteroaryl, wobei C₁-C₆-Alkyl gegebenenfalls mit Aryl substituiert ist,
R⁷ Wasserstoff, C₁-C₆-Alkyl, C₃-C₈-Cycloalkyl, 5- bis 6-gliedriges Heterocyclyl, Aryl oder 5- bis 6-gliedriges Heteroaryl, wobei C₁-C₆-Alkyl gegebenenfalls mit Aryl substituiert ist,
R⁸ C₃-C₈-Cycloalkyl, C₁-C₆-Alkyl oder Phenyl, wobei C₁-C₆-Alkyl mit C₁-C₆-Alkoxy und Phenyl mit 1 bis 3 Resten unabhängig voneinander ausgewählt aus der Gruppe Halogen, Cyano, C₁-C₆-Alkyl, C₁-C₆-Alkoxy, Trifluormethyl und Trifluormethoxy substituiert ist,
bedeutet, und deren Salze, Solvate und Solvate der Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die erfindungsgemäßen Verbindungen können auch in Form ihrer Salze, Solvate oder Solvate der Salze vorliegen.

Als Salze sind im Rahmen der Erfmdung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder N-Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten im Allgemeinen die folgende Bedeutung:
Aryl steht für Naphthyl oder Phenyl, bevorzugt Phenyl.
C₁-C₆- und C₁-C₄-Alkoxy steht für einen geradkettigen oder verzweigten Alkoxyrest mit 1 bis 6 bzw. 1 bis 4, bevorzugt mit 1 bis 4 und besonders bevorzugt mit 1 bis 3 Kohlenstoffatomen. Beispielsweise und vorzugsweise seien genannt: Methoxy, Ethoxy, n-Propoxy, Isopropoxy, tert.-Butoxy, n-Pentoxy und n-Hexoxy.
C₁-C₆- und C₁-C₄-Alkyl stehen für einen geradkettigen oder verzweigten Alkylrest mit 1 bis 6 bzw. 1 bis 4, bevorzugt mit 1 bis 4 und besonders bevorzugt mit 1 bis 3 Kohlenstoffatomen. Beispielsweise und vorzugsweise seien genannt: Methyl, Ethyl, n-Propyl, Isopropyl, tert.-Butyl, n-Pentyl und n-Hexyl.
C₃-C₈-, C₃-C₆- und C₅-C₆-Cycloalkyl stehen für Cyclopropyl, Cyclopentyl, Cyclobutyl, Cyclohexyl, Cycloheptyl oder Cyclooctyl. Bevorzugt seien Cyclopropyl, Cyclopentyl und Cyclohexyl und besonders bevorzugt Cyclopentyl und Cyclohexyl genannt.
Halogen steht für Fluor, Chlor, Brom und Jod. Bevorzugt sind Fluor, Chlor und Brom. Besonders bevorzugt sind Fluor und Chlor.
5- bis 6-gliedriges Heteroaryl steht für einen aromatischen Rest mit 5 bis 6 Ringatomen und bis zu 4, vorzugsweise bis zu 2 Heteroatomen aus der Reihe S, O und/oder N. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Imidazolyl, Pyridyl, Pyrimidinyl, und Pyridazinyl.
5- bis 6-gliedriges Heterocyclyl steht für einen heterocyclischen Rest mit 5 bis 6 Ringatomen und bis zu 3, vorzugsweise 2 Heteroatomen bzw. Heterogruppen aus der Reihe N, O, S, SO, SO₂, bevorzugt sind N und O. Die Heterocyclyl-Reste können gesättigt oder teilweise ungesättigt sein. Gesättigte Heterocyclyl-Reste sind bevorzugt. Die Heterocyclyl-Reste können über ein Kohlenstoffatom oder ein Heteroatom gebunden sein. Beispielsweise und vorzugsweise seinen genannt: Pyrrolinyl, Tetrahydrofuranyl, Tetrahydrothienyl, Pyranyl, Piperidinyl, Piperazinyl, Thiopyranyl, Morpholinyl.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der Formel (I),
in welcher
- R¹: eine Gruppe der Formel -NR²-CO-NR³R⁴, -NR²-CO-CO-OR⁵, -NH-SO₂-R⁶, -SO₂NHR⁷ oder -NH-CO-R⁸, wobei
R² Wasserstoff oder C₁-C₄-Alkyl,
R³ und R⁴ unabhängig voneinander Wasserstoff, C₁-C₄-Alkyl, C₃-C₆-Cycloalkyl oder Phenyl,
welches gegebenenfalls mit bis zu 2 Resten unabhängig voneinander ausgewählt aus der Gruppe Fluor, Chlor, Brom, Cyano, C₁-C₄-Alkyl, C₁-C₄-Alkoxy, Trifluormethyl und Trifluormethoxy substituiert ist, oder
R³ und R⁴ zusammen mit dem Stickstoffatom, an dem sie gebunden sind,
ein 5- bis 6-gliedriges Heterocyclyl bilden,
R⁵ Wasserstoff, C₁-C₄-Alkyl, C₃-C₆-Cycloalkyl, oder Aryl, wobei C₁-C₄-Alkyl gegebenenfalls mit Aryl substituiert ist,
R⁶ C₁-C₄-Alkyl, C₃-C₆-Cycloalkyl, 5- bis 6-gliedriges Heterocyclyl, Aryl oder 5- bis 6-gliedriges Heteroaryl, wobei C₁-C₄-Alkyl gegebenenfalls mit Aryl substituiert ist,
R⁷ Wasserstoff, C₁-C₄-Alkyl, C₃-C₆-Cycloalkyl, 5- bis 6-gliedriges Heterocyclyl, Aryl oder 5- bis 6-gliedriges Heteroaryl, wobei C₁-C₄-Alkyl gegebenenfalls mit Aryl substituiert ist,
R⁸ C₃-C₆-Cycloalkyl, C₁-C₄-Alkyl oder Phenyl, wobei C₁-C₄-Alkyl mit C₁-C₄-Alkoxy und Phenyl mit 1 bis 2 Resten unabhängig voneinander ausgewählt aus der Gruppe Fluor, Chlor, Brom, Cyano, C₁-C₄-Alkyl, C₁-C₄-Alkoxy, Trifluormethyl und Trifluormethoxy substituiert ist,
bedeutet, sowie deren Salze, Solvate und Solvate der Salze.

Ebenfalls bevorzugt sind Verbindungen der Formel (I), in welcher
- R¹: eine Gruppe der Formel -NH-CO-NHR³, -NH-CO-CO-OH, -NH-SO₂-R⁶, -SO₂NHR⁷ oder -NH-CO-R⁸, wobei
R³ Wasserstoff, C₁-C₄-Alkyl, C₅-C₆-Cycloalkyl oder Phenyl,
welches gegebenenfalls mit C₁-C₄-Alkoxy substituiert ist,
R⁶ C₁-C₄-Alkyl oder Phenyl, wobei C₁-C₄-Alkyl gegebenenfalls mit Phenyl substituiert ist,
R⁷ Wasserstoff oder C₁-C₄-Alkyl, welches gegebenenfalls mit Phenyl substituiert ist,
R⁸ C₅-C₆-Cycloalkyl, Methoxymethyl oder Phenyl, welches durch Fluor oder Chlor substituiert ist,
bedeutet, sowie deren Salze, Solvate und Solvate der Salze.

Ebenfalls bevorzugt sind Verbindungen der Formel 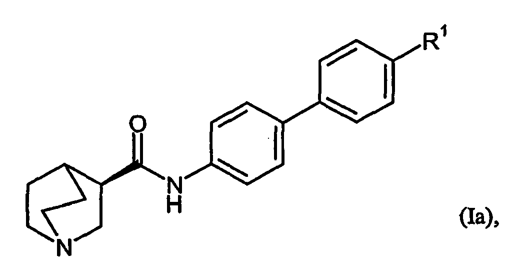 in welcher R¹ die oben angegebenen Bedeutungen aufweist sowie deren Salze, Solvate und Solvate der Salze.

Ganz besonders bevorzugt sind Kombinationen von zwei oder mehreren der oben genannten Vorzugsbereiche.

Die Erfindung betrifft weiterhin Verfahren zur Herstellung der erfindungsgemäßen Verbindungen, dadurch gekennzeichnet, dass man
[A] Verbindungen der Formel 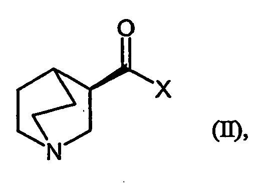 in welcher
   - X: für Hydroxy oder eine geeignete Abgangsgruppe wie beispielsweise Chlor oder Pentafluorphenoxy steht,
   mit einer Verbindung der Formel 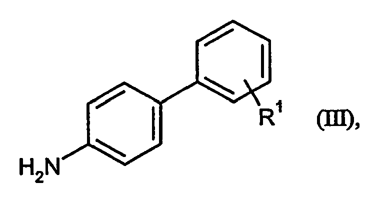 in welcher
   R¹ die oben angegebenen Bedeutungen aufweist,
   in einem inerten Lösungsmittel gegebenenfalls in Gegenwart eines Kondensationsmittels und gegebenenfalls in Gegenwart einer Base umsetzt,
   oder
[B] Verbindungen der Formel (II) zunächst mit einer Verbindung der Formel 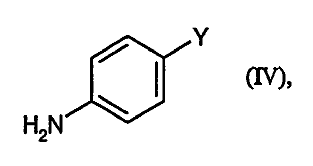 in welcher
   - Y: für eine geeignete Abgangsgruppe wie beispielsweise Triflat oder Halogen, vorzugsweise Brom oder Iod, steht,
   gegebenenfalls in einem inerten Lösungsmittel gegebenenfalls in Gegenwart eines Kondensationsmittels und gegebenenfalls in Gegenwart einer Base zu Verbindungen der Formel 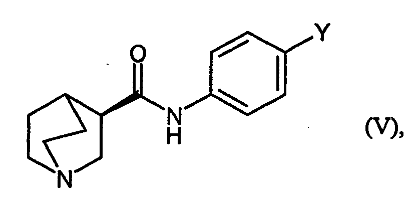 in welcher
   - Y: die oben angegebenen Bedeutungen aufweist,
   umsetzt,
   und diese dann in einer Kupplungsreaktion mit Verbindungen der Formel 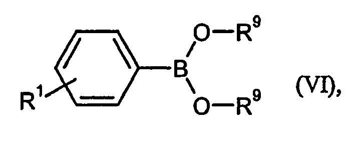 in welcher
   - R¹: die oben angegebenen Bedeutungen aufweist, und
   - R⁹: für Wasserstoff oder Methyl steht oder beide Reste zusammen eine CH₂CH₂- oder C(CH₃)₂-C(CH₃)₂-Brücke bilden,
   in einem inerten Lösungsmittel in Gegenwart eines geeigneten Katalysators und in Gegenwart einer Base umsetzt, und die resultierenden erfindungsgemäßen Verbindungen gegebenenfalls mit den entsprechenden (i) Lösungsmitteln und/oder (ii) Basen oder Säuren zu ihren Solvaten, Salzen und/oder Solvaten der Salze umsetzt.

Wenn X eine Abgangsgruppe ist, sind Chlor, Mesyloxy und Isobutyloxycarbonyloxy, besonders Chlor bevorzugt.

Inerte Lösungsmittel für die Verfahrensschritte (II) + (III) → (I) und (II) + (IV) → (V) sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, Dimethylsulfoxid, Acetonitril oder Pyridin. Bevorzugt ist Dimethylformamid, Tetrahydrofuran, Methylenchlorid oder Chloroform.

Kondensationsmittel für die Verfahrensschritte (II) + (III) → (I) und (II) + (IV) → (V) sind beispielsweise Carbodiimide wie z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodümid-Hydrochlorid (EDC), N-Cyclohexylcarbodümid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformiat, oder Bis-(2-oxo-3-oxazolidinyl)-phosphorylchlorid oder Benzotriazolyloxy-tri(dimethylamino)phosphonium-hexafluorophosphat, oder O-(Benzotriazol-1-yl)-N,N,N',N'-tetramethyluronium-hexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyluronium-hexafluorophosphat (HATU) oder Benzotriazol-l-yloxytris(dimethylamino)-phosphonium-hexafluorophosphat (BOP), oder Mischungen aus diesen.

Gegebenenfalls kann es vorteilhaft sein, diese Kondensationsmittel in Gegenwart eines Hilfsnucleophils wie z.B. 1-Hydroxybenzotriazol (HOBt) zu verwenden.

Besonders bevorzugt ist HATU oder die Kombination von N-(3-Dimethylaminoisopropyl)-N'-ethylcarbodiimid-Hydrochlorid (EDC) und 1-Hydroxybenzotriazol (HOBt) in Dimethylformamid.

Basen für die Verfahrensschritte (II) + (III) → (I) und (II) + (IV) → (V) sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Vorzugsweise werden die Verfahrensschritte (II) + (III) → (I) und (II) + (IV) → (V) in einem Temperaturbereich von Raumtemperatur bis 50°C bei Normaldruck durchgeführt.

Inerte Lösungsmittel für den Verfahrensschritt (V) + (VI) → (I) sind beispielsweise Ether wie Dioxan, Tetrahydrofuran oder 1,2-Dimethoxyethan, Kohlenwasserstoffe wie Benzol, Xylol oder Toluol, oder andere Lösemittel wie Nitrobenzol, Dimethylformamid, Dimethylacetamid, Dimethylsulfoxid oder N-Methylpyrrolidon. Bevorzugt sind Lösungsmittel wie z.B. Dimethylformamid, Dimethylacetamid, Dimethylsulfoxid oder 1,2-Dimethoxyethan.

Für den Verfahrensschritt (V) + (VI) → (I) geeignete Katalysatoren sind beispielsweise für Suzuki-Kupplungen übliche Palladium-Katalysatoren, bevorzugt sind Katalysatoren wie z.B. Dichlorbis(triphenylphosphin)palladium, Tetrakistriphenylphosphinpalladium, Palladium(II)acetat oder Bis-(diphenylphosphino)ferrocen-palladium(II)chlorid (vgl. z.B. A. Suzuki, *Acc. Chem. Res.* **1982,** *15,* 178ff; Miyaura et al., *J. Am. Chem. Soc.* **1989,** *111,* 314).

Für den Verfahrensschritt (V) + (VI) → (I) geeignete Basen sind beispielsweise Kaliumacetat, Cäsium-, Kalium- oder Natriumcarbonat, Bariumhydroxid, Kalium-tert.-butylat, Cäsiumfluorid oder Kaliumphosphat. Bevorzugt ist Cäsiumcarbonat oder Natriumcarbonat.

Vorzugsweise wird der Verfahrensschritt (V) + (VI) → (I) in einem Temperaturbereich von Raumtemperatur bis 130°C bei Normaldruck durchgeführt.

Die Verbindungen der allgemeinen Formeln (II) und (VI) sind bekannt oder lassen sich nach bekannten Verfahren aus den entsprechenden Edukten synthetisieren [vgl. z.B. für Verbindungen der allgemeinen Formel (II): Kato et al., *Chem. Pharm. Bull.* **1995**, *43,* 1351-1357; Orlek et al., *J. Med. Chem.* **1991**, *34*, 2726-2735; Plate et al., *Bioorg. Med. Chem.* **2000**, *8,* 449-454; für Verbindungen der allgemeinen Formel (VI): D.S. Matteson, in: *Stereodirected Synthesis with Organoboranes,* Hrsg. K. Hafner, C.W. Rees, B.M. Trost, J.-M. Lehn, P. v. Ragué Schleyer, Springer-Verlag, Heidelberg 1995; H.C. Brown, G.W. Kramer, A.B. Levy, M.M. Midland, *Organic Synthesis via Boranes,* Wiley, New York 1975; A. Pelter, K. Smith, H.C. Brown, *Borane Reagents,* Academic Press, London 1988].

Die Verbindungen der Formeln (III) und (IV) sind ebenfalls bekannt oder lassen sich nach bekannten Verfahren aus den entsprechenden Edukten synthetisieren (vgl. z.B. *Comprehensive Heterocyclic Chemistry,* Katritzky et al., Hrsg., Elsevier, 1996). So können beispielsweise Benzoesäurederivate gemäß folgendem Syntheseschema via Umlagerung (Curtius-Abbau) der korrespondierenden Carbonsäureazide in die entsprechenden Anilinderivate überführt werden (vgl. z.B. S. Deprets, G. Kirsch, *Eur. J. Org. Chem.* **2000**, *7*, 1353ff.):

### Syntheseschema

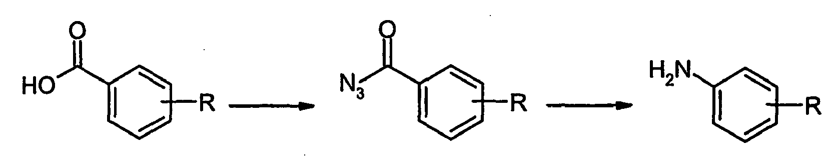

Die erfindungsgemäßen Verbindungen eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspektrum.

Sie zeichnen sich als Liganden, insbesondere als Agonisten am α7-nAChR aus.

Die erfindungsgemäßen Verbindungen können aufgrund ihrer pharmakologischen Eigenschaften allein oder in Kombination mit anderen Arzneimitteln zur Behandlung und/oder Prävention von kognitiven Störungen, insbesondere der Alzheimer'schen Krankheit eingesetzt werden. Wegen ihrer selektiven Wirkung als α7-nAChR-Agonisten eignen sich die erfmdungsgemäßen Verbindungen besonders zur Verbesserung der Wahrnehmung, Konzentrationsleistung, Lernleistung, oder Gedächtnisleistung insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Attention Deficit Hyperactivity Disorder, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrophe Lateralsklerose (ALS), Huntington'sche Krankheit, Multiple Sklerose, Thalamische Degeneration, Creutzfeld-Jacob-Demenz, HIV-Demenz, Schizophrenie, Schizophrenie mit Demenz oder Korsakoff-Psychose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Die erfmdungsgemäßen Verbindungen können allein oder in Kombination mit anderen Wirkstoffen zur Prävention und Behandlung der Folgen von neurodegenerativen Erkrankungen eingesetzt werden. Als bevorzugte Beispiele für neurodegenerative Erkrankungen seien Alzheimer'sche Krankheit und Parkinson'sche Krankheit genannt.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994), insbesondere zur Behandlung von Krebs-induzierten Schmerzen und chronischen neuropathischen Schmerzen, wie zum Beispiel bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Die *in vitro*-Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine Bindungstest ist eine Modifikation der von Davies et al. (*Neuropharmacol.* **1999,** *38,* 679-690) beschriebenen Methode.

Rattenhirngewebe (Hippocampus oder Gesamthirn) wird in Homogenisierungspuffer (10 % w/v) [0.32 M Sucrose, 1 mM EDTA, 0.1 mM Phenylmethylsulfonylfluorid (PMSF), 0.01 % (w/v) NaN₃, pH 7.4, 4°C] bei 600 rpm in einem Glashomogenisator homogenisiert. Das Homogenat wird zentrifugiert (1000 x g, 4°C, 10 min) und der Überstand wird abgenommen. Das Pellet wird erneut suspendiert (20 % w/v) und zentrifugiert (1000 x g, 4°C, 10 min). Die beiden Überstände werden vereinigt und zentrifugiert (15.000 x g, 4°C, 30 min). Dieses Pellet wird als P2-Fraktion bezeichnet.

Das P2-Pellet wird zweimal mit Bindungspuffer gewaschen (50 mM Tris-HCl, 1 mM MgCl₂, 120 mM NaCl, 5 mM KCl, 2 mM CaCl₂, pH 7.4) und zentrifugiert (15.000 x g, 4°C, 30 min).

Die P2-Membranen werden in Bindungspuffer resuspendiert und in einem Volumen von 250 µl (Membranproteinmenge 0.1-0.5 mg) für 2.5 h bei 21°C inkubiert in der Gegenwart von 1-5 nM [³H]-Methyllycaconitine, 0.1 % (w/v) BSA (bovines Serumalbumin) und verschiedenen Konzentrationen der Testsubstanz. Die unspezifische Bindung wird bestimmt durch Inkubation in der Gegenwart von 1 µM □-Bungarotoxin oder 100 µM Nicotin oder 10 µM MLA (Methyllycaconitine).

Die Inkubation wird beendet durch Zugabe von 4 ml PBS (20 mM Na₂HPO₄, 5 mM KH₂PO₄, 150 mM NaCl, pH 7.4, 4°C) und Filtration durch Typ A/E glass fibre filters (Gelman Sciences), die vorher 3 h in 0.3 % (v/v) Polyethylenimin (PEI) eingelegt waren. Die Filter werden zweimal mit 4 ml PBS (4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50% des am Rezeptor gebundenen Liganden verdrängt werden), der Dissoziationskonstante K_{D} und der Konzentration L von [³H]-Methyllycaconitine wird die Dissoziationskonstante der Testsubstanz K; bestimmt (Kᵢ = IC₅₀/ (1+L/K_{D})).

Anstelle von [³H]-Methyllycaconitine können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Repräsentative in-vitro-Wirkdaten für die erfindungsgemäßen Verbindungen sind in Tabelle A wiedergegeben:

**Tabelle A**

| **Beispiel-Nr.** | **Kᵢ-Wert [nM]** |
|---|---|
| 2 | 2 |
| 5 | < 1 |
| 7 | < 1 |
| 11 | 16 |
| 15 | 27 |

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wird wie beschrieben durchgeführt (Blokland et al., *NeuroReport* **1998**, *9*, 4205-4208; Ennaceur, A., Delacour, J., *Behav. Brain Res.* **1988**, *31*, 47-59; Ennaceur, A., Meliani, K., *Psychopharmacology* **1992,** *109,* 321-330; Prickaerts et al., *Eur. J. Pharmacol.* **1997,** *337,* 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest:

Der Soziale Wiedererkennungstest ist ein Test zur Prüfung der lern- oder gedächtnisverbessernden Wirkung von Testsubstanzen.

Erwachsene Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die totale Zeit gemessen, die das adulte Tier das Junge investigiert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das Juvenile herausgenommen und das Adulte in seinem Testkäfig belassen (bei 24 Stunden Retention wird das Tier in seinen Heimkäfig zurückgesetzt). Vor oder nach dem ersten Test wird das Versuchstier mit Substanz behandelt. Je nach Zeitpunkt der Substanzgabe wird das Erlernen oder das Speichern der Information über das Jungtier durch die Substanz beeinflusst. Nach einem festgelegten Zeitraum (Retention) wird der Test wiederholt (Trial 2). Je größer die Differenz zwischen den in Trial 1 und 2 ermittelten Investigationszeiten, desto besser hat sich das adulte Tier an das Jungtier erinnert

Die erfindungsgemäßen Verbindungen eignen sich zur Verwendung als Arzneimittel für Menschen und Tiere.

Zur vorliegenden Erfmdung gehören auch pharmazeutische Zubereitungen, die neben inerten, nicht-toxischen, pharmazeutisch geeigneten Hilfs- und Trägerstoffen eine oder mehrere der erfmdungsgemäßen Verbindungen enthalten, oder die aus einem oder mehreren der erfindungsgemäßen Verbindungen bestehen, sowie Verfahren zur Herstellung dieser Zubereitungen.

Die erfindungsgemäßen Verbindungen sollen in diesen Zubereitungen in einer Konzentration von 0,1 bis 99,5 Gew.-%, bevorzugt von 0,5 bis 95 Gew.-% der Gesamtmischung vorhanden sein.

Neben den erfmdungsgemäßen Verbindungen können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- DAD: Dioden-Array-Detektor
- DCI: direkte chemische Ionisation (bei MS)
- DMF: *N,N*-Dimethylformamid
- DMSO: Dimethylsulfoxid
- d. Th.: der Theorie (bei Ausbeute)
- ESI: Elektrospray-Ionisation (bei MS)
- h: Stunde(n)
- HPLC: Hochdruck- / Hochleistungsflüssigchromatographie
- konz.: konzentriert
- LC-MS: Flüssigchromatographie mit gekoppelter Massenspektroskopie
- min.: Minute(n)
- MS: Massenspektroskopie
- NMR: Kernresonanzspektroskopie
- PBS: phosphate buffered saline
- PdCl₂(dppf): Bis-(diphenylphosphanferrocenyl)-palladium(II)chlorid
- RT: Raumtemperatur (20°C)
- Rₜ: Retentionszeit (bei HPLC)

### HPLC- und LC-MS-Methoden:

### Methode 1:

Instrument: HP 1100 mit DAD-Detektion; Säule: Kromasil RP-18, 60 mm x 2 mm, 3.5 µm; Eluent A: 5 mL HClO₄/L H₂O, Eluent B: Acetonitril; Gradient: 0 min 2 % B, 0.5 min 2 % B, 4.5 min 90 % B, 6.5 min 90 % B; Fluss: 0.75 mL/min; Temperatur: 30°C; UV-Detektion: 210 nm.

### Methode 2:

Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05 % Ameisensäure, Eluent A: Wasser + 0.05 % Ameisensäure; Gradient: 0.0 min 5 % B → 2.0 min 40 % B → 4.5 min 90 % B → 5.5 min 90 % B; Ofen: 45°C; Fluss: 0.0 min 0.75 mL/min → 4.5 min 0.75 mL/min → 5.5 min 1.25 mL/min; UV-Detektion: 210 nm.

### Methode 3:

Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µL 50 %-ige Ameisensäure / L, Eluent B: Acetonitril + 500 µL 50 %-ige Ameisensäure / L; Gradient: 0.0 min 0 % B → 2.9 min 70 % B → 3.1 min 90 % B → 4.5 min 90 % B; Ofen: 50°C; Fluss: 0.8 mL/min; UV-Detektion: 210 nm.

### Ausgangsverbindungen:

### Beispiel 1A

### (rac)-1-Azabicyclo[2.2.2]octan-3-carbonitril

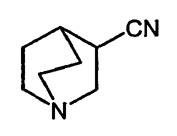

20.4 g (163 mmol) 3-Chinuclidinon und 41.4 g (212 mmol) (4-Toluolsulfonyl)methylisocyanid werden in 435 mL 1,2-Dimethoxyethan und 16 mL trockenem Ethanol unter Eiskühlung vorgelegt. Es werden 45.7 g (407 mmol) Kalium-tert.-butoxid langsam zugegeben, so dass die Tempertaur auf maximal 10°C ansteigt. Anschließend wird 2.5 h lang auf 40°C erhitzt. Nach Abkühlung auf RT wird entstandener Feststoff abfiltriert. Das Filtrat wird eingeengt und über neutrales Aluminiumoxid chromatographiert (Laufmittel: Dichlormethan → Essigsäureethylester → Essigsäureethylester/Methanol 50:1). Es werden 22.9 g (quant.) des racemischen Produktes in leicht verunreinigter Form erhalten.

### Beispiel 2A

### (R)-1-Azabicyclo[2.2.2]octan-3-carbonitril

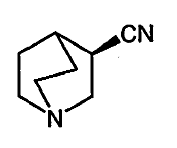

Die Enantiomerentrennung des Racemats aus Beispiel 1A erfolgt mittels HPLC an chiraler Phase [Säule: Daicel Chiralpak AD 250 mm x 20 mm; Eluent: 5 % Wasser, 87 % Acetonitril, 8 % Acetonitril mit 2 % Diethylamin; Fluss: 10 mL/min; Detektion: 220 nm; Injektionsvolumen: 0.3 mL].

Aus der Trennung von 20 g racemischen 1-Azabicyclo[2.2.2]octan-3-carbonitril werden 8.7 g der Titelverbindung (87 % d. Th.) isoliert.

Rₜ = 6.19 min [Chiralpak AD 250 mm x 4.6 mm, 10 µm; Eluent: 5 % Wasser, 95 % Acetonitril mit 2 % Diethylamin; Temperatur: 30°C; Fluss: 1.0 mL/min].

### Beispiel 3A

### (R)-1-Azabicyclo[2.2.2]octan-3-carbonsäure

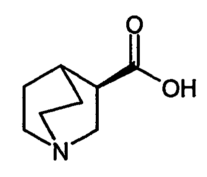

7.50 g (55.1 mmol) (*R*)-1-Azabicyclo[2.2.2]octan-3-carbonitril (Beispiel 2A) werden zusammen mit 78 mL konz. Salzsäure 4 h unter Rückfluss erhitzt. Das Solvens wird unter reduziertem Druck entfernt und verbleibendes Wasser mehrfach mit Toluol abdestilliert. Es werden 12.9 g der Titelsubstanz, die noch anorganische Salze enthält, erhalten und ohne weitere Aufreinigung umgesetzt.

### Beispiel 4A

### (3R)-N-(4-Bromphenyl)-1-azabicyclo[2.2.2]octan-3-carboxamid

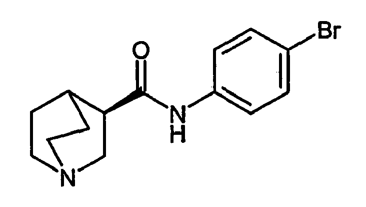

9.17 g (47.8 mmol) (*R*)-1-Azabicyclo[2.2.2]octan-3-carbonsäure (Beispiel 3A) werden zusammen mit 160 mL Thionylchlorid 1 h lang unter Rückfluss erhitzt. Überschüssiges Thionylchlorid wird unter reduziertem Druck entfernt und Reste azeotrop zusammen mit Toluol abdestilliert. Das so erhaltene Säurechlorid wird zusammen mit 8.19 g (47.6 mmol) 4-Bromanilin und 24.6 mL (190.4 mmol) N,N-Diisopropylethylamin in 59 mL DMF 72 h lang bei RT gerührt. Das Solvens wird unter reduziertem Druck entfernt und das Rohprodukt über Kieselgel 60 (Laufmittel: Dichlormethan → Dichlormethan/Methanol/Triethylamin 70:30:2) chromatographisch gereinigt. Die Produktfraktionen werden vereinigt, im Vakuum eingeengt und der Rückstand im Hochvakuum getrocknet. Es werden 5.5 g (37 % d. Th.) der Titelverbindung isoliert. Die absolute Konfiguration wurde durch Kristallstrukturanalyse von Einkristallen zugeordnet.

¹H-NMR (200 MHz, DMSO-d₆): δ = 10.06 (s, 1H), 7.70-7.40 (m, 4H), 3.30-3.10 (m, 1H), 2.94-2.45 (m, 6H), 2.15-2.04 (m, 1H), 1.73-1.45 (m, 3H), 1.45-1.15 (m, 1H).

HPLC (Methode 1): Rₜ= 3.84 min.

MS (ESIpos): m/z = 309 (M+H)⁺.

### Beispiel 5A

### (3R)-Chinuclidin-3-carbonylchlorid-Hydrochlorid

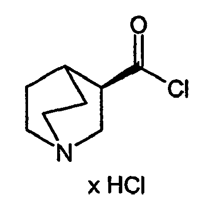

Zu einer Lösung von 2.0 g (12.89 mmol) (*R*)-1-Azabicyclo[2.2.2]octan-3-carbonsäure (Beispiel 3A) in 10 mL Toluol werden 8.18 g (64.43 mmol) Oxalylchlorid zugetropft. Nach 18 h Rühren bei Raumtemperatur wird die Reaktionsmischung im Vakuum eingeengt und zweimal mit Toluol codestilliert. Nach der Trocknung im Hochvakuum erhält man 2.31 g (85.2 % d. Th.) der Titelverbindung, welche ohne weitere Aufreinigung weiter umgesetzt wird.

### Beispiel 6A

### (3R)-N-(4'-Nitrobiphenyl-4-yl)chinuclidin-3-carboxamid-Hydrochlorid

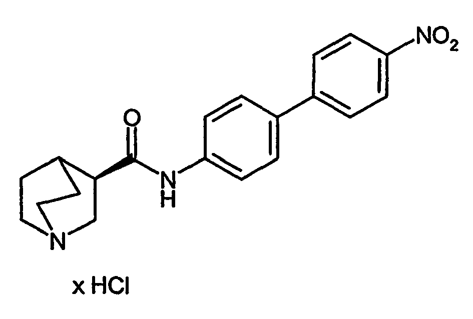

Zu einer unter Argon hergestellten Mischung aus 490 mg (2.33 mmol) (3R)-Chinuclidin-3-carbonylchlorid-Hydrochlorid (Beispiel 5A) und 250 mg (1.17 mmol) 4-Amino-4'-nitrobiphenyl in 11 mL einer 10:1-Mischung aus Dioxan und DMF werden 480 mg (3.50 mmol) Kaliumcarbonat hinzugefügt. Das Reaktionsgemisch wird 18 h lang bei 100°C gerührt und dann eingeengt. Der Rückstand wird in Methanol suspendiert und filtriert. Der Filtrationsrückstand wird mit Wasser gewaschen, mit 20 mL einer 3:1-Mischung aus Acetonitril und 1 N Salzsäure versetzt, erneut eingeengt und im Hochvakuum getrocknet. Das obige Filtrat wird mittels präparativer HPLC aufgereinigt. Die Produktfraktionen werden eingeengt, in 5 mL einer 3: 1-Mischung aus Acetonitril und 1 N Salzsäure aufgenommen, erneut eingeengt und im Hochvakuum getrocknet. Aus dem Filtrationsrückstand werden so 291 mg (61.7 % d. Th.) und aus dem Filtrat weitere 65 mg (12.4 % d. Th.) der Titelverbindung erhalten.

HPLC (Methode 1): Rₜ = 4.13 min.

MS (ESIpos): m/z = 352 (M+H)⁺.

### Beispiel 7A

### (3R)-N-(4'-Aminobiphenyl-4-yl)chinuclidin-3-carboxamid-Dihydrochlorid

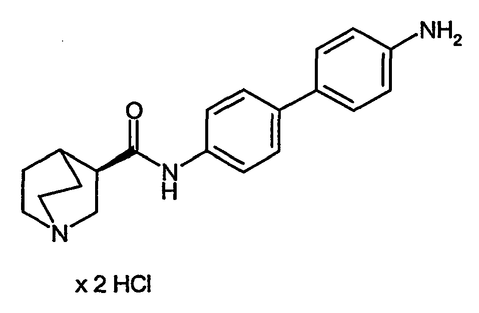

Eine Lösung von 739 mg (2.10 mmol) (3*R*)-N-(4'-Nitrobiphenyl-4-yl)chinuclidin-3-carboxamid (Beispiel 6A) in 10 mL Methanol und 5 mL 2 N Salzsäure wird in Gegenwart von 448 mg (0.21 mmol) 5% Palladium auf Kohle für 2 h unter Normaldruck hydriert. Nach Filtration über Kieselgur wird mit Methanol gewaschen und das Filtrat eingeengt und im Hochvakuum getrocknet. Man erhält 755 mg (89.2 % d. Th.) der Titelverbindung.

HPLC (Methode 1): Rₜ = 3.04 min.

MS (ESIpos): m/z = 322 (M+H)⁺.

### Beispiel 8A

### (3R)-N-(3'-Aminobiphenyl-4-yl)chinuclidin-3-carboxamid-Dihydrochlorid

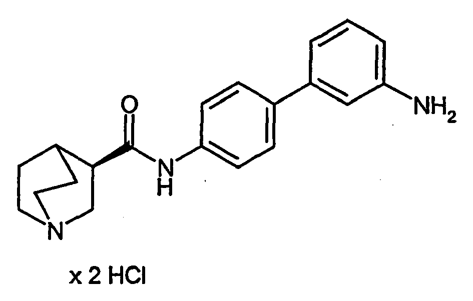

Eine entgaste Mischung aus 200 mg (0.58 mmol) (3*R*)-N-(4-Bromphenyl)-1-azabicyclo-[2.2.2]octan-3-carboxamid (Beispiel 4A), 215.2 mg (0.58 mmol) 3-Aminophenylboronsäure-Hemisulfat, 579 µL (1.74 mmol) 3 N Natronlauge und 21.2 mg (0.03 mmol) PdCl₂(dppf) in 3 mL DMF wird für 18 h auf 90°C erhitzt. Nach dem Abkühlen auf RT wird mittels präparativer HPLC aufgereinigt. Die Produktfraktionen werden eingeengt, mit 3 mL 1 N Salzsäure versetzt und erneut eingeengt. Nach dem Trocknen im Hochvakuum erhält man 146 mg (39.4 % d. Th.) der Titelverbindung, welche ohne weitere Aufreinigung weiter umgesetzt wird.

### Beispiel 9A

### N-Benzyl-4'-nitrobiphenyl-4-sulfonamid

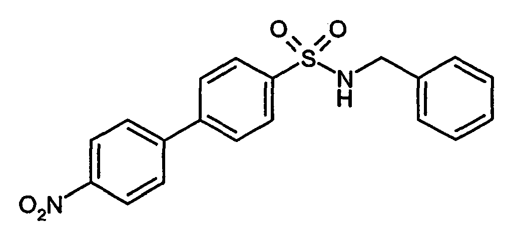

Zu einer Lösung von 150 mg (0.50 mmol) 4'-Nitrobiphenyl-4-sulfonsäurechlorid in 2.0 mL DMF werden 0.28 mL (2.52 mmol) Benzylamin hinzugefügt. Nach 18 h bei Raumtemperatur wird das Reaktionsgemisch mit 2.5 mL Wasser versetzt. Der entstehende Niederschlag wird abgesaugt und im Hochvakuum getrocknet. Man erhält 159 mg (74.4 % d. Th.) der Titelverbindung, welche ohne weitere Reinigung weiter umgesetzt wird.

LC-MS (Methode 3): Rₜ = 3.83 min.; m/z = 369 (M+H)⁺.

### Beispiel 10A

### 4'-Amino-N-benzylbiphenyl-4-sulfonamid

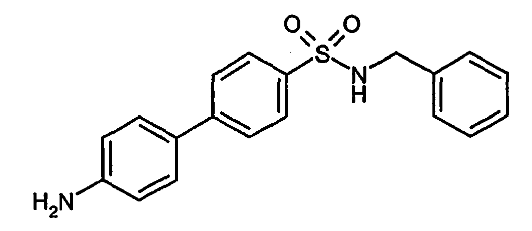

Zu einer Lösung von 136 mg (0.37 mmol) N-Benzyl-4'-nitrobiphenyl-4-sulfonamid (Beispiel 9A) in 2.0 mL DMF werden 416.5 mg (1.85 mmol) Zinn(II)chlorid-Dihydrat hinzugefügt. Nach 18 h bei Raumtemperatur wird das Reaktionsgemisch mittels präparativer HPLC aufgereinigt. Die Produktfraktionen werden im Vakuum eingeengt und im Hochvakuum getrocknet. Man erhält 117 mg (84.3 % d. Th.) der Titelverbindung.

HPLC (Methode 1): Rₜ = 3.91 min.

MS (ESIpos): m/z = 339 (M+H)⁺.

### Beispiel 11A

### N-Isopropyl-4'-nitrobiphenyl-4-sulfonamid

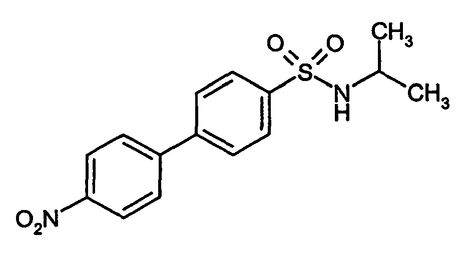

Zu einer Lösung von 150 mg (0.50 mmol) 4'-Nitrobiphenyl-4-sulfonsäurechlorid in 2.0 mL DMF werden 0.22 mL (2.52 mmol) Isopropylamin hinzugefügt. Nach 18 h bei Raumtemperatur wird das Reaktionsgemisch mit 2.5 mL Wasser versetzt. Der entstehende Niederschlag wird abgesaugt und im Hochvakuum getrocknet. Man erhält 126 mg (64.4 % d. Th.) der Titelverbindung, welche ohne weitere Reinigung weiter umgesetzt wird.

LC-MS (Methode 3): Rₜ = 3.66 min.; m/z = 321 (M+H)⁺.

### Beispiel 12A

### 4'-Amino-N-isopropylbiphenyl-4-sulfonamid

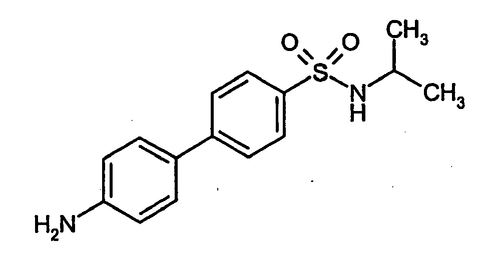

Zu einer Lösung von 100 mg (0.26 mmol) N-Isopropyl-4'-nitrobiphenyl-4-sulfonamid (Beispiel 11A) in 2.0 mL DMF werden 288.8 mg (1.28 mmol) Zinn(II)chlorid-Dihydrat hinzugefügt. Nach 18 h bei Raumtemperatur wird das Reaktionsgemisch mittels präparativer HPLC aufgereinigt. Die Produktfraktionen werden im Vakuum eingeengt und im Hochvakuum getrocknet. Man erhält 47 mg (63.2 % d. Th.) der Titelverbindung.

HPLC (Methode 1): Rₜ = 3.57 min.

MS (DCI): m/z = 291 (M+H)⁺.

### Ausführungsbeispiele:

### Beispiel 1

### [(4'-{[(3R)-1-Azabicyclo[2.2.2]oct-3-ylcarbonyl]amino}biphenyl-4-yl)amino](oxo)essigsäure-Hydrochlorid

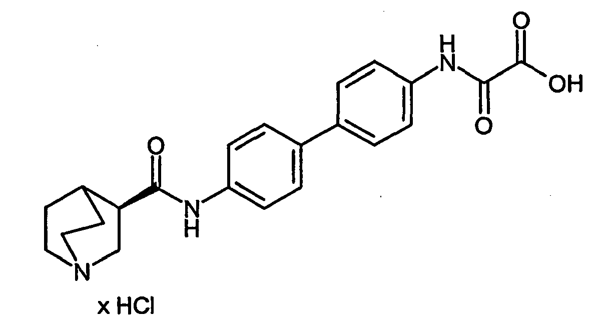

Zu einer unter Argon hergestellten Mischung aus 300 mg (1.43 mmol) (3R)-Chinuclidin-3-carbonylchlorid-Hydrochlorid (Beispiel 5A) und 180 mg (0.71 mmol) [(4'-Aminobiphenyl-4-yl)amino](oxo)essigsäure [CAS Registry-Nr. 100872-66-0] in 11 mL einer 10:1-Mischung aus Dioxan und DMF werden 300 mg (2.14 mmol) Kaliumcarbonat hinzugefügt. Nach 18 h bei 100°C wird das Reaktionsgemisch im Vakuum eingeengt, der Rückstand in Wasser und Acetonitril gelöst und mittels präparativer HPLC aufgereinigt. Die eingeengten Produktfraktionen werden mit 5 mL einer 2:1-Mischung aus Acetonitril und 1 N Salzsäure versetzt und erneut eingeengt. Nach dem Trocknen im Hochvakuum werden 64 mg (20.3 % d. Th.) der Titelverbindung erhalten.

HPLC (Methode 1): Rₜ = 3.40 min.

MS (ESIpos): m/z = 350 (M+H)⁺.

### Beispiel 2

### (3R)-N-{4'-[(Methylsulfonyl)amino]biphenyl-4-yl}chinuclidin-3-carboxamid-Hydrochlorid

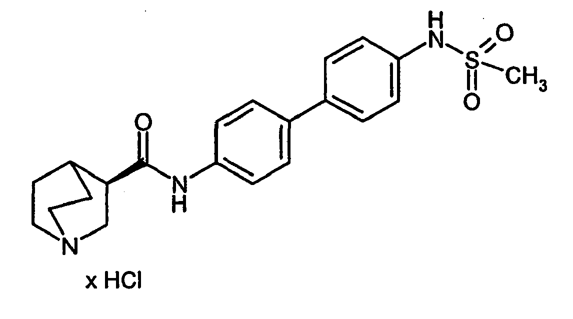

Zu einer unter Argon hergestellten Mischung aus 300 mg (1.43 mmol) (3R)-Chinuclidin-3-carbonylchlorid-Hydrochlorid (Beispiel 5A) und 187 mg (0.71 mmol) N-(4'-aminobiphenyl-4-yl)methansulfonamid [CAS Registry-Nr. 82315-47-7] in 11 mL einer 10:1-Mischung aus Dioxan und DMF werden 300 mg (2.14 mmol) Kaliumcarbonat hinzugefügt. Nach 18 h bei 100°C wird das Reaktionsgemisch im Vakuum eingeengt, der Rückstand in Wasser und Acetonitril gelöst und mittels präparativer HPLC aufgereinigt. Die eingeengten Produktfraktionen werden mit 5 mL einer 2:1-Mischung aus Acetonitril und 1 N Salzsäure versetzt und erneut eingeengt. Nach dem Trocknen im Hochvakuum werden 186 mg (58.3 % d. Th.) der Titelverbindung erhalten.

¹H-NMR (300 MHz, DMSO-d₆): δ = 10.45 (s, 1H), 10.19 (br. s, 1H), 9.80 (s, 1H), 7.71 (m, 2H), 7.61 (m, 4H), 7.28 (m, 2H), 3.60 (dd, 1H), 3.42-3.10 (m, 6H), 3.01 (s, 3H), 2.45 (m, 1H), 1.93 (m, 2H), 1.76 (m, 2H).

HPLC (Methode 1): Rₜ = 3.59 min.

MS (ESIpos): m/z = 400 (M+H)⁺.

### Beispiel 3

### (3R)-N-{3'-[(Methylsulfonyl)amino]biphenyl-4-yl}chinuclidin-3-carboxamid-Hydrochlorid

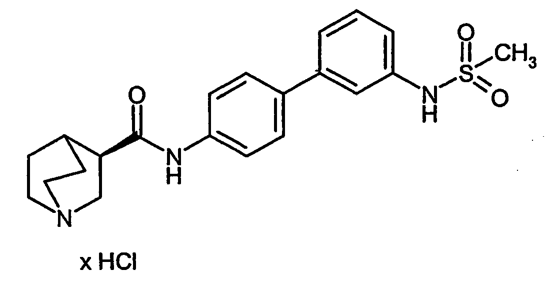

Zu einer Lösung von 60 mg (0.09 mmol) (3*R*)-N-(3'-Aminobiphenyl-4-yl)chinuclidin-3-carboxamid-Dihydrochlorid (Beispiel 8A) in 1 mL DMF werden bei Raumtemperatur 63.6 µL (0.46 mmol) Triethylamin und 21.2 µL (0.27 mmol) Methansulfonsäurechlorid hinzugefügt. Nach 18 h bei Raumtemperatur wird das Reaktionsgemisch mit einer 1:1-Mischung aus Acetonitril und Wasser verdünnt und mittels präparativer HPLC aufgereinigt. Die Produktfraktionen werden eingeengt, in 1 mL 1 N Salzsäure aufgenommen, erneut eingeengt und im Hochvakuum getrocknet. Es werden 14 mg (35.2 % d. Th.) der Titelverbindung erhalten.

HPLC (Methode 1): Rₜ = 3.70 min.

LC-MS (Methode 2): Rₜ = 2.45 min.; m/z = 400 (M+H)⁺.

### Beispiel 4

### (3R)-N-{4'-[(Ethylsulfonyl)amino]biphenyl-4-yl}chinuclidin-3-carboxamid-Hydrochlorid

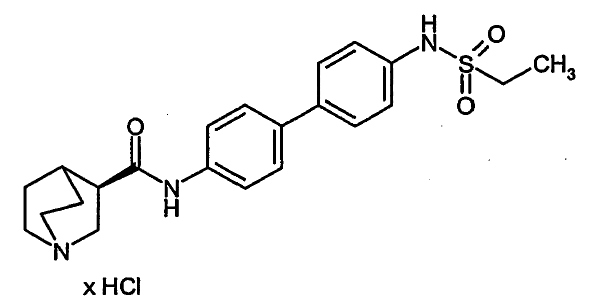

Zu einer Lösung von 60 mg (0.15 mmol) ((3*R*)-N-(4'-Aminobiphenyl-4-yl)chinuclidin-3-carboxamid-Dihydrochlorid (Beispiel 7A) in 0.5 mL DMF werden 39.1 mg (0.30 mmol) Ethansulfonsäurechlorid und 84.8 µL (0.61 mmol) Triethylamin hinzugefügt. Nach 18 h bei Raumtemperatur wird das Reaktionsgemisch mittels präparativer HPLC aufgereinigt. Die Produktfraktionen werden eingeengt, mit 2 mL einer 1: 1 -Mischung aus Acetonitril und 1 N Salzsäure versetzt, erneut eingeengt und im Hochvakuum getrocknet. Man erhält 26 mg (35.2 % d. Th.) der Titelverbindung.

HPLC (Methode 1): Rₜ = 3.71 min.

MS (ESIpos): m/z = 414 (M+H)⁺.

### Beispiel 5

### (3R)-N-{4'-[(Phenylsulfonyl)amino]biphenyl-4-yl}chinuclidin-3-carboxamid-Hydrochlorid

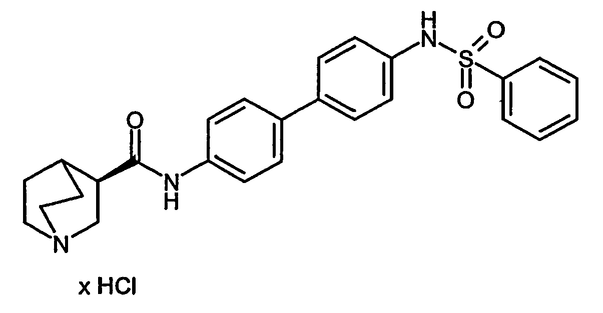

Eine Lösung von 80 mg (0.20 mmol) ((3*R*)-N-(4'-Aminobiphenyl-4-yl)chinuclidin-3-carboxamid-Dihydrochlorid (Beispiel 7A) und 71.7 mg (0.41 mmol) Phenylsulfonsäurechlorid in 1.0 mL Pyridin wird 18 h bei Raumtemperatur gerührt. Das Reaktionsgemisch wird im Vakuum eingeengt und der Rückstand mittels präparativer HPLC aufgereinigt. Die Produktfraktionen werden eingeengt, mit 3 mL 1 N Salzsäure versetzt, erneut eingeengt und im Hochvakuum getrocknet. Man erhält 52 mg (51.5 % d. Th.) der Titelverbindung.

¹H-NMR (400 MHz, DMSO-d₆): δ = 10.40 (s, 1H), 10.37 (s, 1H), 9.85 (br. s, 1H), 7.80 (m, 2H), 7.66 (m, 2H), 7.63-7.49 (m, 7H), 7.17 (m, 2H), 3.61 (dd, 1H), 3.43-3.17 (m, 5H), 3.11 (m, 1H), 2.41 (m, 1H), 1.92 (m, 2H), 1.73 (m, 2H).

HPLC (Methode 1): Rₜ = 4.05 min.

MS (ESIpos): m/z = 462 (M+H)⁺.

### Beispiel 6

### (3R)-N-{4'-[(Benzylsulfonyl)amino]biphenyl-4-yl}chinuclidin-3-carboxamid-Hydrochlorid

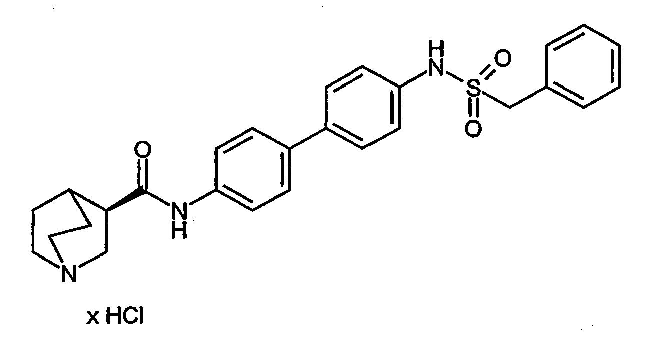

Eine Lösung von 80 mg (0.20 mmol) ((3*R*)-N-(4'-Aminobiphenyl-4-yl)chinuclidin-3-carboxamid-Dihydrochlorid (Beispiel 7A) und 77.4 mg (0.41 mmol) Phenylmethansulfonsäurechlorid in 1.0 mL Pyridin wird 18 h bei Raumtemperatur gerührt. Das Reaktionsgemisch wird im Vakuum eingeengt und der Rückstand mittels präparativer HPLC aufgereinigt. Die Produktfraktionen werden eingeengt, mit 3 mL 1 N Salzsäure versetzt, erneut eingeengt und im Hochvakuum getrocknet. Man erhält 34 mg (32.7 % d. Th.) der Titelverbindung.

HPLC (Methode 1): Rₜ = 4.12 min.

MS (ESIpos): m/z = 476 (M+H)⁺.

### Beispiel 7

### (3R)-N-[4'-(Aminosulfonyl)biphenyl-4-yl]chinuclidin-3-carboxamid-Hydrochlorid

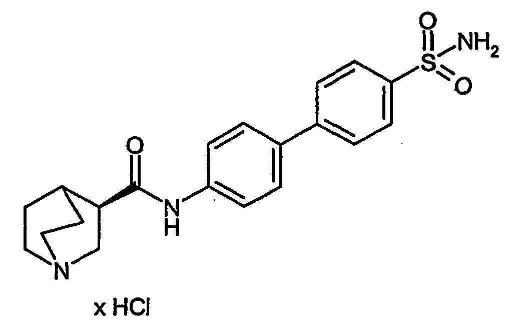

Zu einer unter Argon hergestellten Mischung aus 216.3 mg (1.03 mmol) (3R)-Chinuclidin-3-carbonylchlorid-Hydrochlorid (Beispiel 5A) und 127.8 mg (0.51 mmol) (4'-Amino-4-biphenyl)-sulfonamid in 5.5 mL einer 10:1-Mischung aus Dioxan und DMF werden 213.5 mg (1.54 mmol) Kaliumcarbonat hinzugefügt. Nach 18 h bei 100°C werden weitere 216.3 mg (1.03 mmol) (3R)-Chinuclidin-3-carbonylchlorid-Hydrochlorid hinzugefügt. Nach weiteren 18 h bei 100°C wird das Produkt durch Zugabe von Acetonitril/Wasser (2:1) ausgefällt. Nach dem Trocknen im Hochvakuum werden 148 mg (71.9% d. Th.) der Titelverbindung erhalten.

¹H-NMR (200 MHz, DMSO-d₆): δ = 10.06 (s, 1H), 7.92-7.80 (m, 4H), 7.79-7.68 (m, 4H), 7.39 (s, 2H), 3.22 (dd, 1H), 2.93-2.58 (m, 6H), 2.09 (m, 1H), 1.60 (m, 3H), 1.33 (m, 1H).

HPLC (Methode 1): Rₜ = 3.40 min.

MS (ESIpos): m/z = 386 (M+H)⁺.

### Beispiel 8

### (3R)-N-{4'-[(Isopropylamino)sulfonyl]biphenyl-4-yl}chinuclidin-3-carboxamid-Hydrochlorid

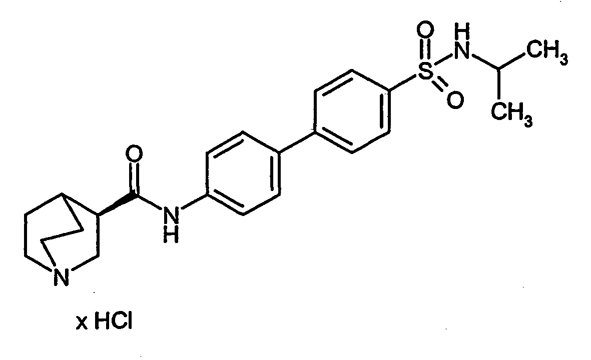

Zu einer unter Argon hergestellten Mischung aus 65.1 mg (0.31 mmol) (3R)-Chinuclidin-3-carbonylchlorid-Hydrochlorid (Beispiel 5A) und 45.0 mg (0.15 mmol) 4'-Amino-N-isopropyl-biphenyl-4-sulfonamid (Beispiel 12A) in 2.2 mL einer 10:1-Mischung aus Dioxan und DMF werden 64.3 mg (0.46 mmol) Kaliumcarbonat hinzugefügt. Nach 18 h bei 100°C wird das Reaktionsgemisch im Vakuum eingeengt. Der Rückstand wird in Wasser und Acetonitril gelöst und mittels präparativer HPLC gereinigt. Die Produktfraktionen werden eingeengt, mit 5 mL einer 2:1-Mischung aus Acetonitril und 1 N Salzsäure versetzt, erneut eingeengt und im Hochvakuum getrocknet. Man erhält 21 mg (29.2 % d. Th.) der Titelverbindung.

HPLC (Methode 1): Rₜ = 3.93 min.

MS (ESIpos): m/z = 428 (M+H)⁺.

### Beispiel 9

### (3R)-N-{4'-[(Benzylamino)sulfonyl]biphenyl-4-yl}chinuclidin-3-carboxamid-Hydrochlorid

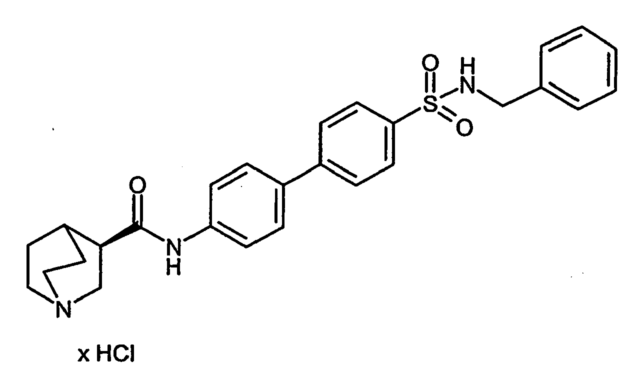

Zu einer unter Argon hergestellten Mischung aus 74.5 mg (0.35 mmol) (3R)-Chinuclidin-3-carbonylchlorid-Hydrochlorid (Beispiel 5A) und 60 mg (0.18 mmol) 4'-Amino-N-benzylbiphenyl-4-sulfonamid (Beispiel 10A) in 2.2 mL einer 10:1-Mischung aus Dioxan und DMF werden 73.5 mg (0.60 mmol) Kaliumcarbonat hinzugefügt. Nach 18 h bei 100°C werden weitere 74.5 mg (0.35 mmol) (3*R*)-Chinuclidin-3-carbonylchlorid-Hydrochlorid hinzugefügt. Nach weiteren 24 h bei 100°C wird das Reaktionsgemisch im Vakuum eingeengt. Der Rückstand wird in Wasser und Acetonitril gelöst und mittels präparativer HPLC gereinigt. Die Produktfraktionen werden eingeengt, mit 5 mL einer 2:1-Mischung aus Acetonitril und 1 N Salzsäure versetzt, erneut eingeengt und im Hochvakuum getrocknet. Man erhält 64 mg (70.5 % d. Th.) der Titelverbindung.

HPLC (Methode 1): Rₜ = 4.17 min.

MS (ESIpos): m/z = 476 (M+H)⁺.

### Beispiel 10

### (3R)-N-(4'-{[(Methylamino)carbonyl]amino}biphenyl-4-yl)chinuclidin-3-carboxamid

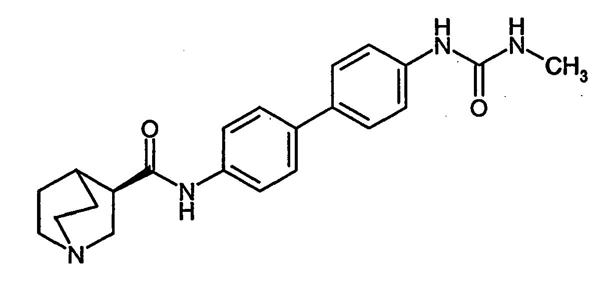

Zu einer Lösung von 60 mg (0.15 mmol) ((3*R*)-N-(4'-Aminobiphenyl-4-yl)chinuclidin-3-carboxamid-Dihydrochlorid (Beispiel 7A) in 0.5 mL DMF werden 17.4 mg (0.30 mmol) Methylisocyanat und 84.8 µL (0.61 mmol) Triethylamin hinzugefügt. Nach 18 h bei Raumtemperatur wird das Reaktionsgemisch mit 5 mL Wasser versetzt. Der entstehende Niederschlag wird abgesaugt, mit Wasser gewaschen und im Hochvakuum getrocknet. Man erhält 47 mg (73.5% d. Th.) der Titelverbindung.

HPLC (Methode 1): Rₜ = 3.44 min.

MS (ESIpos): m/z = 379 (M+H)⁺.

### Beispiel 11

### (3R)-N-(4'-{[(Cyclopentylamino)carbonyl]amino}biphenyl-4-yl)chinuclidin-3-carboxamid-Hydrochlorid

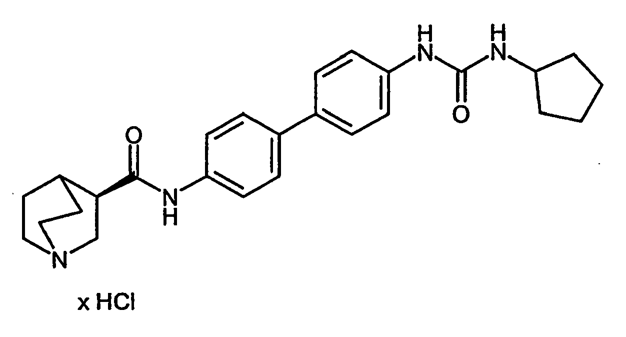

Zu einer Lösung von 60 mg (0.15 mmol) ((3*R*)-N-(4'-Aminobiphenyl-4-yl)chinuclidin-3-carboxamid-Dihydrochlorid (Beispiel 7A) in 0.5 mL DMF werden 33.8 mg (0.30 mmol) Cyclopentylisocyanat und 84.8 µL (0.61 mmol) Triethylamin hinzugefügt. Nach 18 h bei Raumtemperatur wird das Reaktionsgemisch mit 5 mL Wasser versetzt. Der entstehende Niederschlag wird abgesaugt, mit Wasser gewaschen und im Hochvakuum getrocknet. Zur weiteren Aufreinigung wird eine präparative HPLC durchgeführt. Die Produktfraktionen werden eingeengt, mit 3 mL 1 N Salzsäure versetzt, erneut eingeengt und im Hochvakuum getrocknet. Man erhält 28 mg (39.2 % d. Th.) der Titelverbindung.

¹H-NMR (300 MHz, DMSO-d₆): δ = 10.33 (s, 1H), 9.95 (s, 1H), 8.51 (s, 1H), 7.67 (m, 2H), 7.58 (m, 2H), 7.50 (m, 2H), 7.44 (m, 2H), 6.29 (br. s, 1H), 3.96 (m, 1H), 3.60 (m, 1H), 3.37 (m, 1H), 3.29-3.08 (m, 5H), 2.43 (m, 1H), 1.92 (m, 2H), 1.84 (m, 2H), 1.77 (m, 2H), 1.64 (m, 2H), 1.55 (m, 2H), 1.38 (m, 2H).

HPLC (Methode 1): Rₜ = 4.05 min.

MS (ESIpos): m/z = 433 (M+H)⁺.

### Beispiel 12

### (3R)-N-(4'-{[(Ethylamino)carbonyl]amino}biphenyl-4-yl)chinuclidin-3-carboxamid

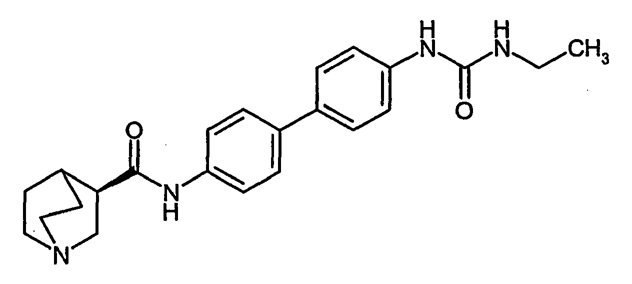

Zu einer Lösung von 60 mg (0.15 mmol) ((3*R*)-N-(4'-Aminobiphenyl-4-yl)chinuclidin-3-carboxamid-Dihydrochlorid (Beispiel 7A) in 0.5 mL DMF werden 21.6 mg (0.30 mmol) Ethylisocyanat und 84.8 µL (0.61 mmol) Triethylamin hinzugefügt. Nach 18 h bei Raumtemperatur wird das Reaktionsgemisch mit 5 mL Wasser versetzt. Der entstehende Niederschlag wird abgesaugt, mit Wasser gewaschen und im Hochvakuum getrocknet. Man erhält 57 mg (88.0% d. Th.) der Titelverbindung.

HPLC (Methode 1): Rₜ = 3.62 min.

MS (ESIpos): m/z = 393 (M+H)⁺.

### Beispiel 13

### (3R)-N-[4'-({[(3-Methoxyphenyl)amino]carbonyl}amino)biphenyl-4-yl]chinuclidin-3-carboxamid-Hydrochlorid

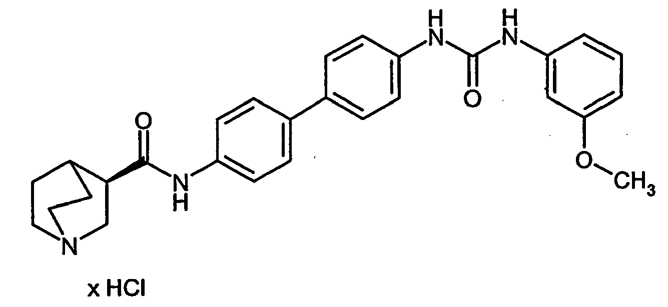

Zu einer Lösung von 60 mg (0.15 mmol) ((3*R*)-N-(4'-Aminobiphenyl-4-yl)chinuclidin-3-carboxamid-Dihydrochlorid (Beispiel 7A) in 0.5 mL DMF werden 45.4 mg (0.30 mmol) 3-Methoxyphenylisocyanat und 84.8 µL (0.61 mmol) Triethylamin hinzugefügt. Nach 18 h bei Raumtemperatur wird das Reaktionsgemisch mit 5 mL Wasser versetzt. Der entstehende Niederschlag wird abgesaugt, mit Wasser gewaschen und im Hochvakuum getrocknet. Zur weiteren Aufreinigung wird eine präparative HPLC durchgeführt. Die Produktfraktionen werden eingeengt, mit 3 mL 1 N Salzsäure versetzt, erneut eingeengt und im Hochvakuum getrocknet. Man erhält 22 mg (27.6% d. Th.) der Titelverbindung.

HPLC (Methode 1): Rₜ = 4.19 min.

MS (ESIpos): m/z = 471 (M+H)⁺.

### Beispiel 14

### (3R)-N-{4'-[(3-Chlorbenzoyl)amino]biphenyl-4-yl}chinuelidin-3-carboxamid-Hydrochlorid

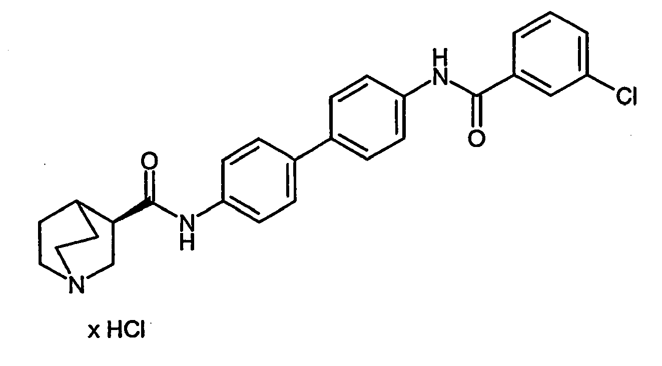

Eine Lösung von 50 mg (0.13 mmol) ((3*R*)-N-(4'-Aminobiphenyl-4-yl)chinuclidin-3-carboxamid-Dihydrochlorid (Beispiel 7A) und 44.4 mg (0.25 mmol) 3-Chlorbenzoesäurechlorid in 1.0 mL Pyridin wird 3 h bei Raumtemperatur gerührt. Das Reaktionsgemisch wird im Vakuum eingeengt und der Rückstand mittels präparativer HPLC aufgereinigt. Die Produktfraktionen werden eingeengt, mit 3 mL 1 N Salzsäure versetzt, erneut eingeengt und im Hochvakuum getrocknet. Man erhält 62 mg (98.5 % d. Th.) der Titelverbindung.

HPLC (Methode 1): Rₜ = 4.41 min.

MS (ESIpos): m/z = 460 (M+H)⁺.

### Beispiel 15

### (3R)-N-{4'-[(3-Fluorbenzoyl)amino]biphenyl-4-yl}chinuclidin-3-carboxamid-Hydrochlorid

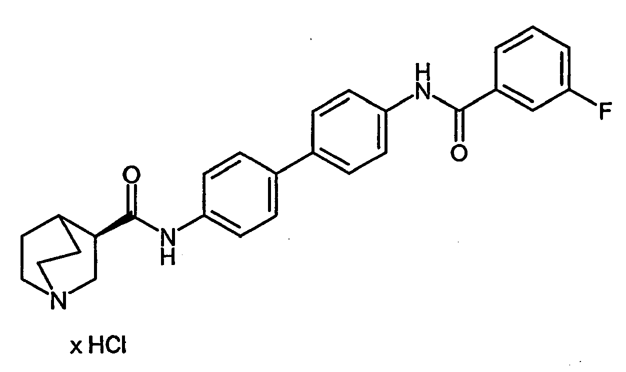

Eine Lösung von 50 mg (0.13 mmol) ((3*R*)-N-(4'-Aminobiphenyl-4-yl)chinuclidin-3-carboxamid-Dihydrochlorid (Beispiel 7A) und 40.2 mg (0.25 mmol) 3-Fluorbenzoesäurechlorid in 1.0 mL Pyridin wird 3 h bei Raumtemperatur gerührt. Das Reaktionsgemisch wird im Vakuum eingeengt und der Rückstand mittels präparativer HPLC aufgereinigt. Die Produktfraktionen werden eingeengt, mit 3 mL 1 N Salzsäure versetzt, erneut eingeengt und im Hochvakuum getrocknet. Man erhält 48 mg (73.4 % d. Th.) der Titelverbindung.

¹H-NMR (200 MHz, DMSO-d₆): δ = 10.48 (s, 1H), 10.43 (s, 1H), 10.21 (br. s, 1H), 7.92-7.80 (m, 3H), 7.79-7.57 (m, 8H), 7.49 (m, 1H), 3.61 (m, 1H), 3.44-3.08 (m, 6H), 2.46 (m, 1H), 1.92 (m, 2H), 1.75 (m, 2H).

HPLC (Methode 1): Rₜ = 4.21 min.

MS (ESIpos): m/z = 444 (M+H)⁺.

### Beispiel 16

### (3R)-N-{4'-[(2-Methoxyacetyl)amino]biphenyl-4-yl}chinuclidin-3-carboxamid-Hydrochlorid

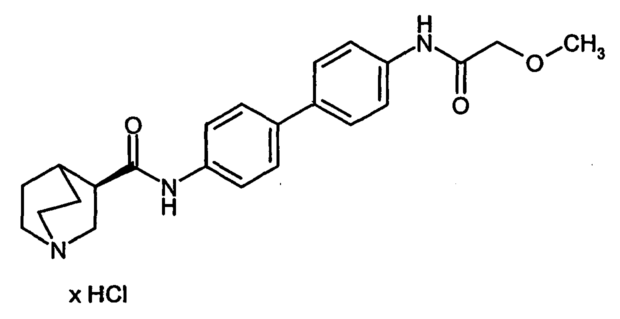

Eine Lösung von 50 mg (0.13 mmol) ((3*R*)-N-(4'-Aminobiphenyl-4-yl)chinuclidin-3-carboxamid-Dihydrochlorid (Beispiel 7A) und 27.5 mg (0.25 mmol) Methoxyessigsäurechlorid in 1.0 mL Pyridin wird 18 h bei Raumtemperatur gerührt. Das Reaktionsgemisch wird mit 3 mL DMSO versetzt und mittels präparativer HPLC aufgereinigt. Die Produktfraktionen werden eingeengt, mit 5 mL 1 N Salzsäure versetzt, erneut eingeengt und im Hochvakuum getrocknet. Man erhält 16 mg (29.4 % d. Th.) der Titelverbindung.

HPLC (Methode 1): Rₜ = 3.63 min.

MS (ESIpos): m/z = 394 (M+H)⁺.

### Beispiel 17

### (3R)-N-{4'-[(Cyclopentylcarbonyl)amino]biphenyl-4-yl}chinuclidin-3-carboxamid-Hydrochlorid

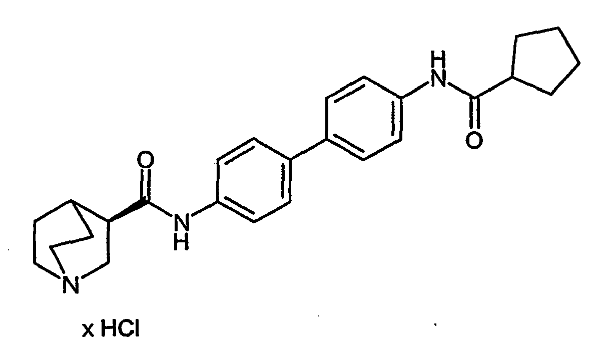

Eine Lösung von 50 mg (0.13 mmol) ((3*R*)-N-(4'-Aminobiphenyl-4-yl)chinuclidin-3-carboxamid-Dihydrochlorid (Beispiel 7A) und 38.5 µL (0.32 mmol) Cyclopentancarbonsäurechlorid in 1.0 mL Pyridin wird 18 h bei Raumtemperatur gerührt. Das Reaktionsgemisch wird mit 3 mL DMSO versetzt und mittels präparativer HPLC aufgereinigt. Die Produktfraktionen werden eingeengt, mit 5 mL 1 N Salzsäure versetzt, erneut eingeengt und im Hochvakuum getrocknet. Man erhält 22 mg (38.2 % d. Th.) der Titelverbindung.

HPLC (Methode 1): Rₜ = 4.18 min.

MS (ESIpos): m/z = 418 (M+H)⁺.

## Patentansprüche

1. Verbindungen der Formel 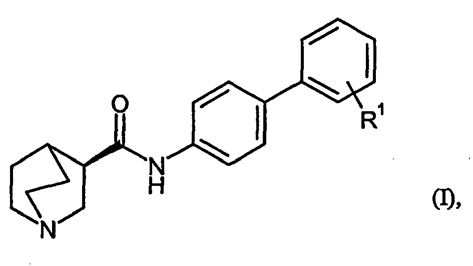 in welcher
R¹ eine Gruppe der Formel -NR²-CO-NR³R⁴, -NR²-CO-CO-OR⁵, -NH-SO₂-R⁶, -SO₂NHR⁷ oder -NH-CO-R⁸, wobei
R² Wasserstoff oder C₁-C₆-Alkyl,
R³ und R⁴ unabhängig voneinander Wasserstoff, C₁-C₆-Alkyl, C₃-C₈-Cycloalkyl oder Phenyl,
welches gegebenenfalls mit bis zu 3 Resten unabhängig voneinander ausgewählt aus der Gruppe Halogen, Cyano, C₁-C₆-Alkyl, C₁-C₆-Alkoxy, Trifluormethyl und Trifluormethoxy substituiert ist, oder
R³ und R⁴ zusammen mit dem Stickstoffatom, an dem sie gebunden sind, ein 5- bis 6-gliedriges Heterocyclyl bilden,
R⁵ Wasserstoff, C₁-C₆-Alkyl, C₃-C₈-Cycloalkyl oder Aryl, wobei C₁-C₆-Alkyl gegebenenfalls mit Aryl substituiert ist,
R⁶ C₁-C₆-Alkyl, C₃-C₈-Cycloalkyl, 5- bis 6-gliedriges Heterocyclyl, Aryl oder 5- bis 6-gliedriges Heteroaryl, wobei C₁-C₆-Alkyl gegebenenfalls mit Aryl substituiert ist,
R⁷ Wasserstoff, C₁-C₆-Alkyl, C₃-C₈-Cycloalkyl, 5- bis 6-gliedriges Heterocyclyl, Aryl oder 5- bis 6-gliedriges Heteroaryl, wobei C₁-C₆-Alkyl gegebenenfalls mit Aryl substituiert ist,
R⁸ C₃-C₈-Cycloalkyl, C₁-C₆-Alkyl oder Phenyl, wobei C₁-C₆-Alkyl mit C₁-C₆-Alkoxy und Phenyl mit 1 bis 3 Resten unabhängig voneinander ausgewählt aus der Gruppe Halogen, Cyano, C₁-C₆-Alkyl, C₁-C₆-Alkoxy, Trifluormethyl und Trifluormethoxy substituiert ist,
bedeutet, und deren Salze, Solvate und Solvate der Salze.

2. Verbindungen nach Anspruch 1, wobei
R¹ eine Gruppe der Formel -NR²-CO-NR³R⁴, -NR²-CO-CO-OR⁵, -NH-SO₂-R⁶, -SO₂NHR⁷ oder -NH-CO-R⁸, wobei
R² Wasserstoff oder C₁-C₄-Alkyl,
R³ und R⁴ unabhängig voneinander Wasserstoff, C₁-C₄-Alkyl, C₃-C₆-Cycloalkyl oder Phenyl,
welches gegebenenfalls mit bis zu 2 Resten unabhängig voneinander ausgewählt aus der Gruppe Fluor, Chlor, Brom, Cyano, C₁-C₄-Alkyl, C₁-C₄-Alkoxy, Trifluormethyl und Trifluormethoxy substituiert ist, oder
R³ und R⁴ zusammen mit dem Stickstoffatom, an dem sie gebunden sind, ein 5- bis 6-gliedriges Heterocyclyl bilden,
R⁵ Wasserstoff, C₁-C₄-Alkyl, C₃-C₆-Cycloalkyl, oder Aryl, wobei C₁-C₄-Alkyl gegebenenfalls mit Aryl substituiert ist,
R⁶ C₁-C₄-Alkyl, C₃-C₆-Cycloalkyl, 5- bis 6-gliedriges Heterocyclyl, Aryl oder 5- bis 6-gliedriges Heteroaryl, wobei C₁-C₄-Alkyl gegebenenfalls mit Aryl substituiert ist,
R⁷ Wasserstoff, C₁-C₄-Alkyl, C₃-C₆-Cycloalkyl, 5- bis 6-gliedriges Heterocyclyl, Aryl oder 5- bis 6-gliedriges Heteroaryl, wobei C₁-C₄-Alkyl gegebenenfalls mit Aryl substituiert ist,
R⁸ C₃-C₆-Cycloalkyl, C₁-C₄-Alkyl oder Phenyl, wobei C₁-C₄-Alkyl mit C₁-C₄-Alkoxy und Phenyl mit 1 bis 2 Resten unabhängig voneinander ausgewählt aus der Gruppe Fluor, Chlor, Brom, Cyano, C₁-C₄-Alkyl, C₁-C₄-Alkoxy, Trifluormethyl und Trifluormethoxy substituiert ist,
bedeutet, sowie deren Salze, Solvate und Solvate der Salze.

3. Verbindungen nach einem der Ansprüche 1 und 2, wobei
R¹ eine Gruppe der Formel -NH-CO-NHR³, NH-CO-CO-OH, NH-SO₂-R⁶, -SO₂NHR⁷ oder-NH-CO-R⁸, wobei
R³ Wasserstoff, C₁-C₄-Alkyl, C₅-C₆-Cycloalkyl oder Phenyl,
welches gegebenenfalls mit C₁-C₄-Alkoxy substituiert ist,
R⁶ C₁-C₄-Alkyl oder Phenyl, wobei C₁-C₄-Alkyl gegebenenfalls mit Phenyl substituiert ist,
R⁷ Wasserstoff oder C₁-C₄-Alkyl, welches gegebenenfalls mit Phenyl substituiert ist,
R⁸ C₅-C₆-Cycloalkyl, Methoxymethyl oder Phenyl, welches durch Fluor oder Chlor substituiert ist,
bedeutet, sowie deren Salze, Solvate und Solvate der Salze.

4. Verbindungen nach einem der Ansprüche 1, 2 oder 3, der Formel 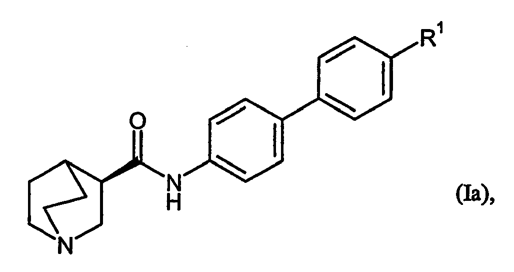 in welcher R¹ die in Anspruch 1 angegebenen Bedeutungen aufweist sowie deren Salze, Solvate und Solvate der Salze.

5. Verfahren zur Herstellung von Verbindungen nach Ansprüchen 1 bis 4, **dadurch gekennzeichnet, dass** man
[A] Verbindungen der Formel 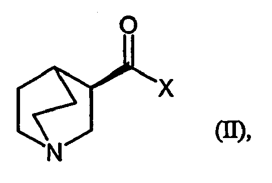 in welcher
X für Hydroxy oder eine geeignete Abgangsgruppe wie beispielsweise Chlor oder Pentafluorphenoxy steht,
mit einer Verbindung der Formel 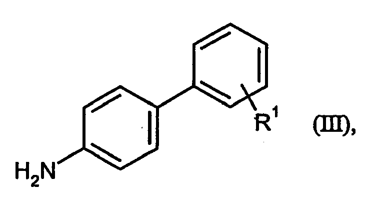 in welcher
R¹ die in Anspruch 1 angegebenen Bedeutungen aufweist,
in einem inerten Lösungsmittel gegebenenfalls in Gegenwart eines Kondensationsmittels und gegebenenfalls in Gegenwart einer Base umsetzt,
oder
[B] Verbindungen der Formel (II) zunächst mit einer Verbindung der Formel 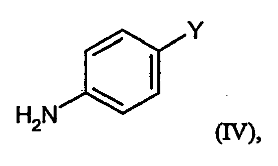 in welcher
Y für eine geeignete Abgangsgruppe wie beispielsweise Triflat oder Halogen, vorzugsweise Brom oder Iod, steht,
gegebenenfalls in einem inerten Lösungsmittel gegebenenfalls in Gegenwart eines Kondensationsmittels und gegebenenfalls in Gegenwart einer Base zu Verbindungen der Formel 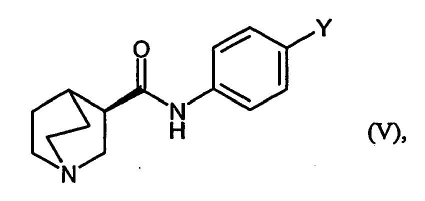 in welcher
Y die oben angegebenen Bedeutungen aufweist,
umsetzt,
und diese dann in einer Kupplungsreaktion mit Verbindungen der Formel 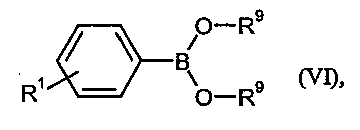 in welcher
R¹ die in Anspruch 1 angegebenen Bedeutungen aufweist, und
R⁹ für Wasserstoff oder Methyl steht oder beide Reste zusammen eine CH₂CH₂- oder C(CH₃)₂-C(CH₃)₂-Brücke bilden,
in einem inerten Lösungsmittel in Gegenwart eines geeigneten Katalysators und in Gegenwart einer Base umsetzt,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

6. Verbindungen nach einem der Ansprüche 1 bis 4 zur Behandlung und/oder Prophylaxe von Krankheiten.

7. Arzneimittel enthaltend mindestens eine der Verbindungen nach einem der Ansprüche 1 bis 4 in Zusammenmischung mit mindestens einem pharmazeutisch verträglichen, im Wesentlichen nichtgiftigen Träger oder Exzipienten.

8. Verwendung von Verbindungen nach einem der Ansprüche 1 bis 4 zur Herstellung eines Arzneimittels zur Verbesserung der Wahrnehmung, Konzentrationsleistung, Lernleistung und/oder Gedächtnisleistung.

9. Verwendung von Verbindungen nach einem der Ansprüche 1 bis 4 zur Herstellung eines Arzneimittels zur Behandlung und/oder Prophylaxe von Störungen der Wahrnehmung, Konzentrationsleistung, Lernleistung und/oder Gedächtnisleistung.

10. Arzneimittel nach Anspruch 7 zur Behandlung und/oder Prophylaxe von Störungen der Wahrnehmung, Konzentrationsleistung, Lernleistung und/oder Gedächtnisleistung.

## Claims

1. Compounds of the formula 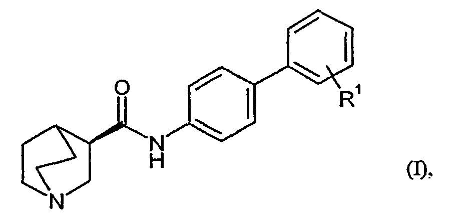 in which
R¹ is a group of the formula -NR²-CO-NR³R⁴, -NR²-CO-CO-OR⁵, -NH- SO₂R⁶, -SO₂NHR⁷ or -NH-CO-R⁸, where
R² is hydrogen or C₁-C₆-alkyl,
R³ and R⁴ are independently of one another hydrogen, C₁-C₆-alkyl, C₃-C₈-cycloalkyl or phenyl, which is optionally substituted by up to 3 radicals independently of one another selected from the group of halogen, cyano, C₁-C₆-alkyl, C₁-C₆-alkoxy, trifluoromethyl and trifluoromethoxy, or
R³ and R⁴ together with the nitrogen atom to which they are bonded form a 5- to 6-membered heterocyclyl,
R⁵ is hydrogen, C₁-C₆-alkyl, C₃-C₈-cycloalkyl or aryl, where C₁-C₆-alkyl is optionally substituted by aryl,
R⁶ is C₁-C₆-alkyl, C₃-C₈-cycloalkyl, 5- to 6-membered heterocyclyl, aryl or 5- to 6-membered heteroaryl, where C₁-C₆-alkyl is optionally substituted by aryl,
R⁷ is hydrogen, C₁-C₆-alkyl, C₃-C₈-cycloalkyl, 5- to 6-membered heterocyclyl, aryl or 5- to 6-membered heteroaryl, where C₁-C₆-alkyl is optionally substituted by aryl,
R⁸ is C₃-C₈-cycloalkyl, C₁-C₆-alkyl or phenyl, where C₁-C₆-alkyl is substituted by C₁-C₆-alkoxy and phenyl by 1 to 3 radicals independently of one another selected from the group of halogen, cyano, C₁-C₆-alkyl, C₁-C₆-alkoxy, trifluoromethyl and trifluoromethoxy,
and the salts, solvates and solvates of the salts thereof.

2. Compounds according to Claim 1, where
R¹ is a group of the formula -NR²-CO-NR³R⁴, -NR²-CO-CO-OR⁵, -NH-SO₂R⁶, -SO₂NHR⁷ or -NH-CO-R⁸, where R² is hydrogen or C₁-C₄-alkyl,
R³ and R⁴ are independently of one another hydrogen, C₁-C₄-alkyl, C₃-C₆-cycloalkyl or phenyl, which is optionally substituted by up to 2 radicals independently of one another selected from the group of fluorine, chlorine, bromine, cyano, C₁-C₄-alkyl, C₁-C₄-alkoxy, trifluoromethyl and trifluoromethoxy, or
R³ and R⁴ together with the nitrogen atom to which they are bonded form a 5- to 6-membered heterocyclyl,
R⁵ is hydrogen, C₁-C₄-alkyl, C₃-C₆-cycloalkyl, or aryl, where C₁-C₄-alkyl is optionally substituted by aryl,
R⁶ is C₁-C₄-alkyl, C₃-C₆-cycloalkyl, 5- to 6-membered heterocyclyl, aryl or 5- to 6-membered heteroaryl, where C₁-C₄-alkyl is optionally substituted by aryl,
R⁷ is hydrogen, C₁-C₄-alkyl, C₃-C₆-cycloalkyl, 5- to 6-membered heterocyclyl, aryl or 5- to 6-membered heteroaryl, where C₁-C₄-alkyl is optionally substituted by aryl,
R⁸ is C₃-C₆-cycloalkyl, C₁-C₄-alkyl or phenyl, where C₁-C₄-alkyl is substituted by C₁-C₄-alkoxy and phenyl by 1 to 2 radicals independently of one another selected from the group of fluorine, chlorine, bromine, cyano, C₁-C₄-alkyl, C₁-C₄-alkoxy, trifluoromethyl and trifluoromethoxy,
and the salts, solvates and solvates of the salts thereof.

3. Compounds according to either of Claims 1 and 2, where
R¹ is a group of the formula -NH-CO-NHR³, -NH-CO-CO-OH, -NH-SO₂R⁶, -SO₂NHR⁷ or -NH-CO-R⁸, where
R³ is hydrogen, C₁-C₄-alkyl, C₅-C₆-cycloalkyl or phenyl, which is optionally substituted by C₁-C₄-alkoxy,
R⁶ is C₁-C₄-alkyl or phenyl, where C₁-C₄-alkyl is optionally substituted by phenyl,
R⁷ is hydrogen or C₁-C₄-alkyl which is optionally substituted by phenyl,
R8 is C₅-C₆-cycloalkyl, methoxymethyl or phenyl which is substituted by fluorine or chlorine,
and the salts, solvates and solvates of the salts thereof.

4. Compounds according to any of Claims 1, 2 or 3, of the formula 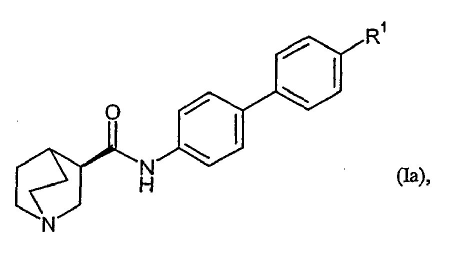 in which R¹ has the meanings indicated in claim 1, and the salts, solvates and solvates of the salts thereof.

5. Process for preparing compounds according to Claims 1 to 4, **characterized in that**
[A] compounds of the formula 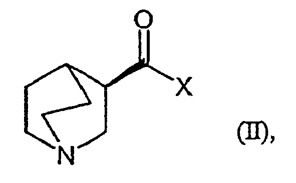 in which
X is hydroxy or a suitable leaving group such as, for example, chlorine or pentafluorophenoxy,
are reacted with a compound of the formula 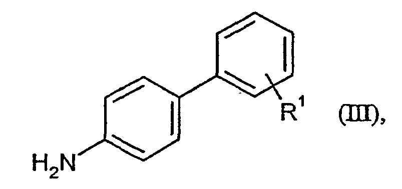 in which
R¹ has the meanings indicated in claim 1, in an inert solvent, where appropriate in the presence of a condensing agent and where appropriate in the presence of a base,
or
[B] compounds of the formula (II) initially are reacted with a compound of the formula 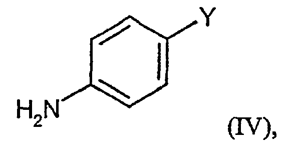
in which
Y is a suitable leaving group such as, for example, triflate or halogen, preferably bromine or iodine,
where appropriate in an inert solvent, where appropriate in the presence of a condensing agent and where appropriate in the presence of a base to give compounds of the formula 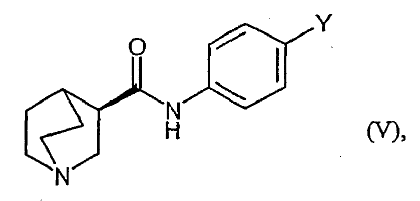 in which
Y has the abovementioned meanings, and the latter are then reacted in a coupling reaction with compounds of the formula 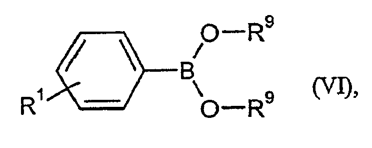
in which
R¹ has the meanings indicated in claim 1, and
R⁹ is hydrogen or methyl, or thet two radicals together form a CH₂CH₂ or C(CH₃)₂-C(CH₃)₂ bridge,
in an inert solvent in the presence of a suitable catalyst and in the presence of a base,
and the resulting compounds of the formula I are reacted where appropriate with the appropriate (i) solvents and/or (ii) bases or acids to give the solvates, salts and/or solvates of the salts thereof.

6. Compounds according to any of Claims 1 to 4 for the treatment and/or prophylaxis of diseases.

7. Medicament comprising at least one of the compounds according to any of Claims 1 to 4 mixed with at least one pharmaceutically acceptable, essentially nontoxic carrier or excipient.

8. Use of compounds according to any of Claims 1 to 4 for producing a medicament for improving perception, concentration, learning and/or memory.

9. Use of compounds according to any of Claims 1 to 4 for producing a medicament for the treatment and/or prophylaxis of impairments of perception, concentration, learning and/or memory.

10. Medicament according to Claim 7 for the treatment and/or prophylaxis of impairments of perception, concentration, learning and/or memory.

## Revendications

1. Composés de formule 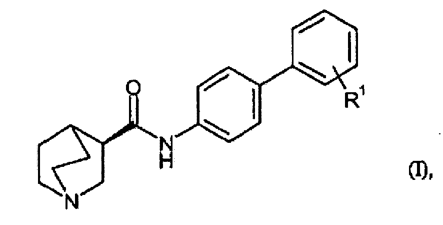 dans laquelle
R¹ désigne un groupe de formule -NR²-CO-NR³R⁴, -NR²-CO-CO-OR⁵, -NH-SO²-R⁶, -SO₂NHR⁷ ou -NH-CO-R⁸, où
R² représente l'hydrogène ou un reste alkyle en C₁ à C₆,
R³ et R⁴ représentent, indépendamment l'un de l'autre, l'hydrogène, un reste alkyle en C₁ à C₆, cycloalkyle en C₃ à C₈ ou phényle,
qui est éventuellement substitué avec jusqu'à 3 restes choisis, indépendamment les uns des autres, dans le groupe des restes halogéno, cyano, alkyle en C₁ à C₆, alkoxy en C₁ à C₆, trifluorométhyle et trifluorométhoxy,
ou bien
R³ et R⁴ forment, conjointement avec l'atome d'azote auquel ils sont liés, un reste hétérocyclyle pentagonal ou hexagonal,
R⁵ représente l'hydrogène, un reste alkyle en C₁ à C₆, cycloalkyle en C₃ à C₈ ou un reste aryle, le reste alkyle en C₁ à C₆ étant éventuellement substitué avec un radical aryle,
R⁶ représente un reste alkyle en C₁ à C₆, cycloalkyle en C₃ à C₈, hétérocyclyle pentagonal ou hexagonal, aryle ou hétéroaryle pentagonal ou hexagonal, le reste alkyle en C₁ à C₆ étant éventuellement substitué avec un radical aryle,
R⁷ représente l'hydrogène, un reste alkyle en C₁ à C₆, cycloalkyle en C₃ à C₈, hétérocyclyle pentagonal ou hexagonal, aryle ou hétéroaryle pentagonal ou hexagonal, le reste alkyle en C₁ à C₆ étant éventuellement substitué avec un radical aryle,
R⁸ représente un reste cycloalkyle en C₃ à C₈, alkyle en C₁ à C₆ ou phényle, le reste alkyle en C₁ à C₆ étant substitué avec un radical alkoxy en C₁ à C₆ et le reste phényle étant substitué avec 1 à 3 restes choisis, indépendamment les uns des autres, dans le groupe des restes halogéno, cyano, alkyle en C₁ à C₆, alkoxy en C₁ à C₆, trifluorométhyle et trifluorométhoxy,
et leurs sels, leurs produits de solvatation et les produits de solvatation de leurs sels.

2. Composés suivant la revendication 1, dans lesquels
R¹ désigne un groupe de formule -NR²-CO-NR³R⁴, -NR²-CO-CO-OR⁵, -NH-SO²-R⁶, -SO₂NHR⁷ ou -NH-CO-R⁸, où
R² représente l'hydrogène ou un reste alkyle en C₁ à C₄,
R³ et R⁴ représentent, indépendamment l'un de l'autre, l'hydrogène, un reste alkyle en C₁ à C₄, cycloalkyle en C₃ à C₆ ou phényle,
qui est éventuellement substitué avec jusqu'à 2 restes choisis, indépendamment l'un de l'autre, dans le groupe des restes fluoro, chloro, bromo, cyano, alkyle en C₁ à C₄, alkoxy en C₁ à C₄, trifluorométhyle et trifluorométhoxy,
ou bien
R³ et R⁴ forment, conjointement avec l'atome d'azote auquel ils sont liés, un reste hétérocyclyle pentagonal ou hexagonal,
R⁵ représente l'hydrogène, un reste alkyle en C₁ à C₄, cycloalkyle en C₃ à C₆ ou aryle, le reste alkyle en C₁ à C₄ étant éventuellement substitué avec un radical aryle,
R⁶ représente un reste alkyle en C₁ à C₄, cycloalkyle en C₃ à C₆, hétérocyclyle pentagonal ou hexagonal, aryle ou hétéroaryle pentagonal ou hexagonal, le reste alkyle en C₁ à C₄ étant éventuellement substitué avec un radical aryle,
R⁷ représente l'hydrogène, un reste alkyle en C₁ à C₄, cycloalkyle en C₃ à C₆, hétérocyclyle pentagonal ou hexagonal, aryle ou hétéroaryle pentagonal ou hexagonal, le reste alkyle en C₁ à C₄ étant éventuellement substitué avec un radical aryle,
R⁸ représente un reste cycloalkyle en C₃ à C₆, alkyle en C₁ à C₄ ou phényle, le reste alkyle en C₁ à C₄ étant substitué avec un radical alkoxy en C₁ à C₄ et le reste phényle étant substitué avec 1 ou 2 restes choisis, indépendamment l'un de l'autre, dans le groupe des restes fluoro, chloro, bromo, cyano, alkyle en C₁ à C₄, alkoxy en C₁ à C₄, trifluorométhyle et trifluorométhoxy,
ainsi que leurs sels, leurs produits de solvatation et les produits de solvatation des sels.

3. Composés suivant l'une des revendications 1 et 2, dans lesquels
R¹ désigne un groupe de formule -NH-CO-NHR³, -NH-CO-CO-OH, -NH-SO₂-R⁶, -SO₂NHR⁷ ou -NH-CO-R⁸, où
R³ représente l'hydrogène, un reste alkyle en C₁ à C₄, cycloalkyle en C₅ ou C₆ ou phényle, qui est éventuellement substitué avec un radical alkoxy en C₁ à C₄,
R⁶ représente un reste alkyle en C₁ à C₄, ou phényle, le reste alkyle en C₁ à C₄ étant éventuellement substitué avec un radical phényle,
R⁷ représente l'hydrogène ou un reste alkyle en C₁ à C₄, qui est éventuellement substitué avec un radical phényle,
R⁸ représente un reste cycloalkyle en C₅ ou C₆, méthoxyméthyle ou phényle, qui est substitué par du fluor ou du chlore,
ainsi que leurs sels, leurs produits de solvatation et les produits de solvatation des sels.

4. Composés suivant l'une des revendications 1, 2 ou 3, de formule 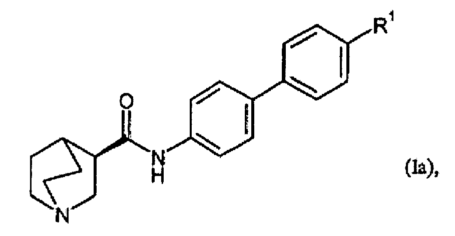 dans laquelle R¹ a les définitions indiquées dans la revendication 1, ainsi que leurs sels, leurs produits de solvatation et les produits de solvatation des sels.

5. Procédé de production de composés suivant les revendications 1 à 4, **caractérisé en ce que**
[A] on fait réagir des composés de formule 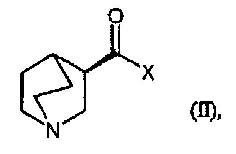 dans laquelle
X représente un groupe hydroxy ou un groupe partant convenable tel que, par exemple, chlore ou pentafluorophénoxy,
avec un composé de formule 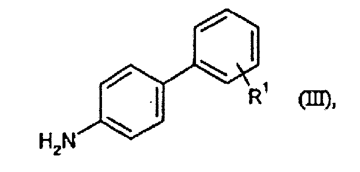 dans laquelle
R¹ a les définitions indiquées dans la revendication 1, dans un solvant inerte, éventuellement en présence d'un agent de condensation et, le cas échéant, en présence d'une base,
ou bien
[B] on fait tout d'abord réagir des composés de formule (II) avec un composé de formule 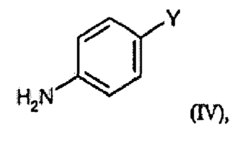 dans laquelle
Y est un groupe partant convenable tel que par exemple triflate ou halogène, avantageusement brome ou iode,
le cas échéant en présence d'un solvant inerte et, éventuellement, en présence d'un agent de condensation, et le cas échéant en présence d'une base, pour obtenir des composés de formule 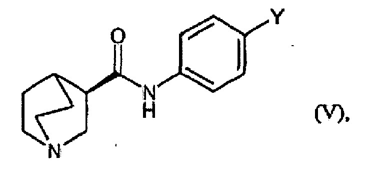 dans laquelle
Y a les définitions indiquées ci-dessus,
puis on fait réagir ces composés dans une réaction de couplage avec des composés de formule 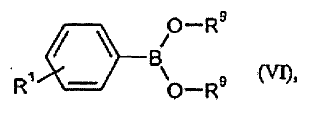 dans laquelle
R¹ a les définitions indiquées dans la revendication 1 et
R⁹ représente l'hydrogène ou le reste méthyle, ou bien les deux restes forment conjointement un pont CH₂CH₂- ou C(CH₃)₂-C(CH₃)₂-,
dans un solvant inerte en présence d'un catalyseur
convenable et en présence d'une base,
et on transforme éventuellement les composés résultants de formule (I) avec (i) les solvants correspondants et/ou (ii) les bases ou les acides correspondants, leurs produits de solvatation, leurs sels et/ou les produits de solvatation des sels.

6. Composés suivant l'une des revendications 1 à 4, destinés au traitement et/ou à la prophylaxie de maladies.

7. Médicament contenant au moins l'un des composés suivant l'une des revendications 1 à 4 en combinaison avec au moins un support ou excipient essentiellement non toxique, pharmaceutiquement acceptable.

8. Utilisation de composés suivant l'une des revendications 1 à 4 pour la préparation d'un médicament destiné à améliorer la perception, la capacité de concentration, la capacité d'apprentissage et/ou la capacité de mémoire.

9. Utilisation de composés suivant l'une des revendications 1 à 4 pour la préparation d'un médicament destiné au traitement et/ou à la prophylaxie de troubles de la perception, de la capacité de concentration, de la capacité d'apprentissage et/ou la capacité de mémoire.

10. Médicament suivant la revendication 7, destiné au traitement et/ou à la prophylaxie de troubles de la perception, de la capacité de concentration, de la capacité d'apprentissage et/ou la capacité de mémoire.
